# EUROPÄISCHE PATENTSCHRIFT

(11) **EP 1 497 256 B1**
(45) Veröffentlichungstag und Bekanntmachung des Hinweises auf die Patenterteilung: **09.06.2010**
(21) Anmeldenummer: 02795123.5
(22) Anmeldetag: 09.12.2002
(51) Int. Cl.: C07C 215/18, C07C 215/20, C07C 215/30, A61K 31/133, A61P 23/02, A61P 9/06, A61P 1/08, A61P 29/00, A61P 37/08, A61P 9/02, A61P 1/04, A61P 25/06, A61P 11/06, A61P 27/06, A61P 25/30

(54) **SUBSTITUIERTE 1,5-DIAMINOPENTAN-3-OL-VERBINDUNGEN**
SUBSTITUTED 1,5-DIAMINOPENTAN-3-OL COMPOUNDS
COMPOSES DE 1,5-DIAMINOPENTANE-3-OL SUBSTITUES

(30) Priorität: 14.12.2001 DE 10161818
(43) Veröffentlichungstag der Anmeldung: 19.01.2005
(73) Patentinhaber: Grünenthal GmbH, 52078 Aachen (DE)
(72) Erfinder: URAGG, Heinz, 52223 Stolberg (DE); MAUL, Corinna, 52066 Aachen (DE); BUSCHMANN, Helmut, E-08950 Esplugues de Llobregat (ES); SUNDERMANN, Bernd, 52066 Aachen (DE); ENGLBERGER, Werner, 52223 Stolberg (DE)
(74) Vertreter: Kutzenberger, Helga
(86) Internationale Anmeldenummer: PCT/EP2002/013912
(87) Internationale Veröffentlichungsnummer: WO 2003/051819

(56) Entgegenhaltungen:
- GB-A- 783 627
- F.F.BLICKE ET.AL.: "Disubstitution of ycloalkanones in the Mannich Reaction" J. ORG. CHEM., Bd. 24, 1959, Seiten 1069-1076, XP002238304

## Beschreibung

Die vorliegende Erfindung betrifft substituierte 1,5-Diaminopentan-3-ol-Verbindungen, Verfahren zu deren Herstellung, Arzneimittel enthaltend diese Verbindungen und die Verwendung von substituierten 1,5-Diaminopentan-3-ol-Verbindungen zur Herstellung von Arzneimitteln.

Die Behandlung von Schmerz hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis sehr starker Schmerzen wirksam. Als unerwünschte Nebenwirkungen weisen sie jedoch unter anderem Atemdepression, Erbrechen, Sedierung, Obstipation und Toleranzentwicklung auf. Außerdem sind sie bei neuropathischen oder inzidentiellen Schmerzen, unter denen insbesondere Tumorpatienten leiden, weniger wirksam.

Aufgabe der vorliegenden Erfindung war es daher, neue Verbindungen zur Verfügung zu stellen, die als pharmazeutische Wirkstoffe in Arzneimitteln eingesetzt werden können und sich insbesondere zur Bekämpfung von Schmerz, insbesondere von chronischem und/oder nichtchronischem Schmerz eignen.

Erfindungsgemäß wird diese Aufgabe durch Bereitstellung von substituierten 1,5-Diaminopentan-3-ol-Verbindungen der nachstehenden allgemeinen Formel I gelöst, da diese Verbindungen insbesondere eine ausgeprägte analgetische Wirkung aufweisen und zur Bekämpfung von Schmerz, insbesondere von chronischem und/oder nichtchronischem Schmerz, als Lokalanästhetikum, Antiarrhythmikum, Antiemetikum und/oder Nootropikum (Neurotropikum), zur Behandlung von inflammatorischen und/oder allergischen Reaktionen, cardiovaskulären Erkrankungen, Harninkontinenz, Diarrhöe, Gastritis, Ulcera, Schockzuständen, Migräne, Narkolepsie, Übergewicht, Asthma, Glaukom, Tinnitus, des hyperkinetischen Syndroms, Pruritus, Alkohol- und/oder Drogen- und/oder Medikamentenmißbrauch und/oder -abhängigkeit und/oder Entzündungen und/oder Depressionen und/oder zur Vigilanzsteigerung, zur Libidosteigerung und/oder zur Behandlung von neurodegenerativen Erkankungen, insbesondere Morbus Parkinson und/oder Morbus Huntington, zur Behandlung und/oder Prophylaxe von Epilepsie, Schizophrenie, Morbus Alzheimer, Schlaganfall, cerebraler Ischämie, cerebralem Infarkt, Hirnödem und/oder zur Anxiolyse und/oder zur Anästhesie eingesetzt werden können.

Gegenstand der vorliegenden Erfindung sind daher substituierte 1,5-Diaminopentan-3-ol-Verbindungen der allgemeinen Formel I, 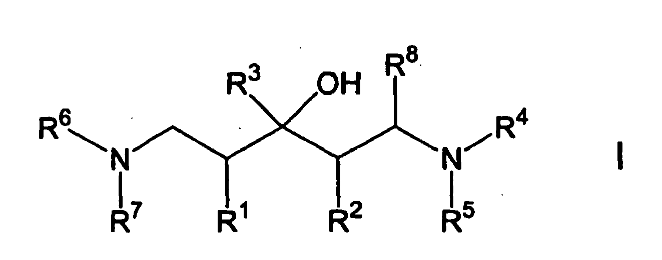 worin
R¹ und R², gleich oder verschieden, jeweils für einen linearen oder verzweigten, gesättigten oder ungesättigten aliphatischen Rest stehen oder zusammen eine (CH₂)ₙ-Kette bilden, wobei n für eine ganze Zahl größer oder gleich 3 steht,
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert und/oder über eine lineare oder verzweigte, gesättigte oder ungesättigte aliphatische Brücke gebunden und/oder der Aryl- oder Heteroarylrest Teil eines polycyclischen Systems sein kann,
R⁴ und R⁵, gleich oder verschieden, jeweils für einen linearen oder verzweigten, gesättigten oder ungesättigten aliphatischen Rest oder einen über eine lineare oder verzweigte, gesättigte oder ungesättigte aliphatische Brücke gebundenen Arylrest stehen oder zusammen eine (CH₂)ₘ-Kette bilden, wobei m für eine ganze Zahl steht,
R⁶ und R⁷, gleich oder verschieden, jeweils für einen linearen oder verzweigten, gesättigten oder ungesättigten aliphatischen Rest oder einen über eine lineare oder verzweigte, gesättigte oder ungesättigte aliphatische Brücke gebundenen Arylrest stehen oder zusammen eine (CH₂)ₚ-Kette bilden, wobei p für eine ganze Zahl steht,
R⁸ für Wasserstoff oder einen gegebenenfalls einfach oder mehrfach substituierten Aryl- oder Heteroarylrest steht, wobei der Aryl- oder Heteroarylrest Teil eines polycyclischen Systems sein kann,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate,
mit Ausnahme der Verbindungen 1,5-Bis-(N,N'-dimethylamino)-2,4-dimethyl-3-pyridin-2-ylpentan-3-ol, 2,6-Bis-[(N,N'-dimethylamino)methyl]-1-phenylcyclohexanol, 2,6-Bis-[(N,N'-dimethylamino)methyl]-1-pyridin-2-ylcyclohexanol und 2,7-Bis-[(N,N'-dimethylamino)methyl]-1-pyridin-2-ylcycloheptanol.

Bevorzugt sind Verbindungen der allgemeinen Formel I, worin
R¹ und R², gleich oder verschieden, jeweils für einen linearen oder verzweigten, gesättigten oder ungesättigten aliphatischen C₁₋₆-Rest stehen oder zusammen eine (CH₂)ₙ-Kette bilden, wobei n für eine ganze Zahl von 3 - 9 steht,
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert und/oder über eine lineare oder verzweigte, gesättigte oder ungesättigte aliphatische C₁₋₅-Brücke gebunden sein kann,
R⁴ und R⁵, gleich oder verschieden, jeweils für einen linearen oder verzweigten, gesättigten oder ungesättigten aliphatischen C₁₋₆-Rest, einen über eine lineare oder verzweigte, gesättigte oder ungesättigte aliphatische C₁₋₅-Brücke gebundenen Phenylrest stehen oder zusammen eine (CH₂)ₘ-Kette bilden, wobei m für eine ganze Zahl von 4 - 10 steht,
R⁶ und R⁷, gleich oder verschieden, jeweils für einen linearen oder verzweigten, gesättigten oder ungesättigten aliphatischen C₁₋₆-Rest, einen über eine lineare oder verzweigte, gesättigte oder ungesättigte aliphatische C₁₋₅-Brücke gebundenen Phenylrest stehen oder zusammen eine (CH₂)ₚ-Kette bilden, wobei p für eine ganze Zahl von 4 - 10 steht,
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate,
mit Ausnahme der Verbindungen 1,5-Bis-(N,N'-dimethylamino)-2,4-dimethyl-3-pyridin-2-ylpentan-3-ol, 2,6-Bis-[(N,N'-dimethylamino)methyl]-1-phenylcyclohexanol, 2,6-Bis-[(N,N'-dimethylamino)methyl]-1-pyridin-2-ylcyclohexanol und 2,7-Bis-[(N,N'-dimethylamino)methyl]-1-pyridin-2-ylcycloheptanol.

Weiter bevorzugt sind Verbindungen der allgemeinen Formel I, worin
R¹ und R², gleich oder verschieden, jeweils für einen linearen oder verzweigten, gesättigten oder ungesättigten aliphatischen C₁₋₃-Rest stehen oder zusammen eine (CH₂)ₙ-Kette bilden, wobei n für eine ganze Zahl von 3 - 5 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe mit vorzugsweise 1 bis 6 Kohlenstoffatomen, einer Alkoxygruppe mit vorzugsweise 1 bis 6 Kohlenstoffatomen und/oder einer trihalogenierten Alkylgruppe, vorzugsweise einer trihalogenierten Methylgruppe, substituiert und/oder über eine lineare oder verzweigte, gesättigte oder ungesättigte aliphatische C₁₋₃-Brücke gebunden sein kann,
R⁴ und R⁵, gleich oder verschieden, jeweils für einen linearen oder verzweigten, gesättigten oder ungesättigten aliphatischen C₁₋₃-Rest stehen oder zusammen eine (CH₂)ₘ-Kette bilden, wobei m für eine ganze Zahl von 4 - 6 steht,
R⁶ und R⁷, gleich oder verschieden, jeweils für einen linearen oder verzweigten, gesättigten oder ungesättigten aliphatischen C₁₋₃-Rest stehen oder zusammen eine (CH₂)ₚ-Kette bilden, wobei p für eine ganze Zahl von 4 - 6 steht,
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate,
mit Ausnahme der Verbindungen 1,5-Bis-(N,N'-dimethylamino)-2,4-dimethyl-3-pyridin-2-ylpentan-3-ol, 2,6-Bis-[(N,N'-dimethylamino)methyl]-1-phenylcyclohexanol, 2,6-Bis-[(N,N'-dimethylamino)methyl]-1-pyridin-2-ylcyclohexanol und 2,7-Bis-[(N,N-dimethylamino)methyl]-1-pyridin-2-ylcycloheptanol.

Weiter bevorzugt sind Verbindungen der allgemeinen Formel I, worin
R¹ und R² zusammen eine (CH₂)ₙ-Kette bilden, wobei n für 3 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann,
R⁴ und R⁵ zusammen eine (CH₂)ₘ-Kette bilden, wobei m für 5 steht,
R⁶ und R⁷ zusammen eine (CH₂)ₚ-Kette bilden, wobei p für 5 steht,
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Besonders bevorzugt sind Verbindungen der allgemeinen Formel II, 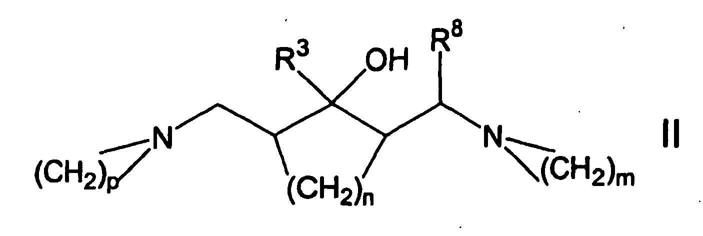 worin
m und p, gleich oder verschieden, für eine ganze Zahl von 4-10 stehen,
n für eine ganze Zahl größer oder gleich 3 steht,
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert und/oder über eine lineare oder verzweigte, gesättigte oder ungesättigte aliphatische Brücke gebunden und/oder der Aryl- oder Heteroarylrest Teil eines polycyclischen Systems sein kann und
R⁸ für Wasserstoff oder einen gegebenenfalls einfach oder mehrfach substituierten Aryl- oder Heteroarylrest steht, wobei der Aryl- oder Heteroarylrest Teil eines polycyclischen Systems sein kann,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Weiter bevorzugt sind Verbindungen der allgemeinen Formel II, worin
m und p, gleich oder verschieden, für eine ganze Zahl von 4-10 stehen,
n für eine ganze Zahl von 3-9 steht und
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann,
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Weiter bevorzugt sind Verbindungen der allgemeinen Formel II, worin
m und p, gleich oder verschieden, für eine ganze Zahl von 4-6 stehen,
n für eine ganze Zahl von 3-5 steht und
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe mit vorzugsweise 1 bis 6 Kohlenstoffatomen, einer Alkoxygruppe mit vorzugsweise 1 bis 6 Kohlenstoffatomen und/oder einer trihalogenierten Alkylgruppe, vorzugsweise einer trihalogenierten Methylgruppe, substituiert und/oder über eine lineare oder verzweigte, gesättigte oder ungesättigte aliphatische C₁₋₃-Brücke gebunden sein kann,
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Weiter bevorzugt sind Verbindungen der allgemeinen Formel II, worin
m und p für 5 stehen,
n für 3 steht und
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann, und
R⁸ für Wasserstoff steht,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Unter einem Heteroarylrest wird ein gegebenenfalls ein- oder mehrfach substituierter, fünf- oder sechsgliedriger aromatischer Rest mit mindestens einem, gegebenenfalls 2, 3, 4 oder 5 Heteroatomen, die gleich oder verschieden sein können, verstanden, der Teil eines polycylischen Systems sein kann. Bevorzugte Heteroatome sind Stickstoff, Sauerstoff und Schwefel. Besonders bevorzugt sind Heteroarylreste ausgewählt aus der Gruppe umfassend Pyrrolyl-, Indolyl-, Furyl- (Furanyl-), Benzofuranyl-, Thienyl- (Thiophenyl-), Benzothienyl-, Pyrazolyl-, Imidazolyl-, Thiazolyl-, Oxazolyl-, Isoxazoyl-, Pydridinyl-, Pyridazinyl-, Pyrimidinyl-, Pyrazinyl-, Pyranyl-, Indazolyl-, Purinyl-, Indolizinyl-, Chinolinyl-, Isochinolinyl-, Chinazolinyl-, Carbazolyl-, Phenazinyl- und Phenothiazinylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Unter einem Acylrest wird ein gegebenenfalls ein- oder mehrfach substituierter, aromatischer Rest verstanden, der Teil eines polycylischen Systems sein kann. Besonders bevorzugt ist ein Phenylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Ganz besonders bevorzugt sind Verbindungen ausgewählt aus der Gruppe umfassend
1-Phenyl-2,6-bis-piperidin-1-ylmethyl-cyclohexanol,
1-(4-Chlor-phenyl)-2,6-bis-piperidin-1-ylmethyl-cyclohexanol,
1-Benzyl-2,6-bis-piperidin-1-ylmethyl-cyclohexanol,
1-(4-Fluor-3-methyl-phenyl)-2,6-bis-piperidin-1-ylmethyl-cyclohexanol,
2,6-Bis-piperidin-1-ylmethyl-1-o-tolyl-cyclohexanol,
2,6-Bis-piperidin-1-ylmethyl-1-vinyl-cyclohexanol,
1-(4-tert-Butyl-phenyl)-2,6-bis-piperidin-1-ylmethyl-cyclohexanol,
1-Cyclopentyl-2,6-bis-piperidin-1-ylmethyl-cyclohexanol,
2,6-Bis-piperidin-1-ylmethyl-1-m-tolyl-cyclohexanol,
2,6-Bis-piperidin-1-ylmethyl-bicyclohexyl-1-ol,
1-(4-Fluor-phenyl)-2,6-bis-piperidin-1-ylmethyl-cyclohexanol,
1-Phenethyl-2,6-bis-piperidin-1-ylmethyl-cyclohexanol,
1-Phenylethynyl-2,6-bis-piperidin-1-ylmethyl-cyclohexanol,
2,6-Bis-piperidin-1-ylmethyl-1-thiophen-2-yl-cyclohexanol,
1-(2,4-Dichlor-phenyl)-2,6-bis-piperidin-1-ylmethyl-cyclohexanol,
1-(3-Methoxy-phenyl)-2,6-bis-piperidin-1-ylmethyl-cyclohexanol,
1-(3-Phenyl-propyl)-2,6-bis-piperidin-1-ylmethyl-cyclohexanol,
1-(2,3-Dichlor-phenyl)-2,6-bis-piperidin-1-ylmethyl-cyclohexanol,
2,6-Bis-piperidin-1-ylmethyl-1-p-tolyl-cyclohexanol,
1-(4-Methoxy-phenyl)-2,6-bis-piperidin-1-ylmethyl-cyclohexanol,
1-Cyclohexylmethyl-2,6-bis-piperidin-1-ylmethyl-cyclohexanol,
1-(5-Fluor-2-methoxy-phenyl)-2,6-bis-piperidin-1-ylmethyl-cyclohexanol,
1-(3-Fluor-phenyl)-2,6-bis-piperidin-1-ylmethyl-cyclohexanol,
1-(3-Chlor-phenyl)-2,6-bis-piperidin-1-ylmethyl-cyclohexanol,
1-(3,5-Dichlor-phenyl)-2,6-bis-piperidin-1-ylmethyl-cyclohexanol,
1-(2-Chlor-benzyl)-2,6-bis-piperidin-1-ylmethyl-cyclohexanol,
1-(4-Fluor-benzyl)-2,6-bis-piperidin-1-ylmethyl-cyclohexanol,
1-(3-Methoxy-benzyl)-2,6-bis-piperidin-1-ylmethyl-cyclohexanol,
1-(4-Chlor-3-trifluormethyl-phenyl)-2,6-bis-piperidin-1-ylmethyl-cyclohexanol,
1-(3-Fluor-benzyl)-2,6-bis-piperidin-1-ylmethyl-cyclohexanol,
1-(2-Methoxy-phenyl)-2,6-bis-piperidin-1-ylmethyl-cyclohexanol,
1-(2-Methyl-benzyl)-2,6-bis-piperidin-1-ylmethyl-cyclohexanol,
1-(3-Chlor-4-fluor-phenyl)-2,6-bis-piperidin-1-ylmethyl-cyclohexanol,
2,6-Bis-piperidin-1-ylmethyl-1-(3-trifluormethyl-phenyl)-cyclohexanol,
1-(3-Methyl-benzyl)-2,6-bis-piperidin-1-ylmethyl-cyclohexanol,
1-(4-Chlor-benzyl)-2,6-bis-piperidin-1-ylmethyl-cyclohexanol,
1-(2-Chlor-6-fluor-benzyl)-2,6-bis-piperidin-1-ylmethyl-cyclohexanol,
1-(2,5-Dimethyl-benzyl)-2,6-bis-piperidin-1-ylmethyl-cyclohexanol,
1-(3-Chlor-benzyl)-2,6-bis-piperidin-1-ylmethyl-cyclohexanol und
1-(2,4-Dichlor-benzyl)-2,6-bis-piperidin-1-ylmethyl-cyclohexanol,

in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate.

Ein weiterer Gegenstand der vorliegenden Erfindung ist ein Verfahren zur Herstellung von substituierten 1,5-Diaminopentan-3-ol-Verbindungen der allgemeinen Formel I, in dem man
A₁) ein Keton der allgemeinen Formel (1), worin R¹ und R² die vorstehend genannte Bedeutung haben, 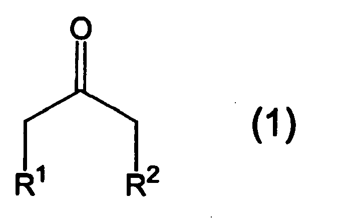 schrittweise mit Paraformaldehyd und jeweils einem Amin der allgemeinen Formel (2) oder (2a), worin R⁴, R⁵, R⁶ und R⁷die vorstehend genannte Bedeutung haben und wobei die beiden Amine der allgemeinen Formeln (2) und (2a) vorzugsweise identisch sind, 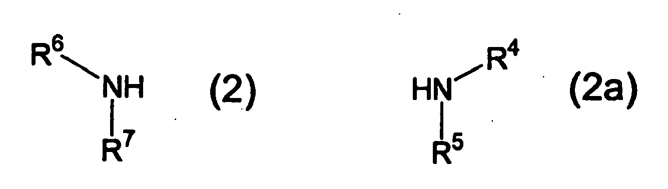 gemäß einer Mannich-Reaktion in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der allgemeinen Formel (3) isoliert und gegebenenfalls reinigt oder 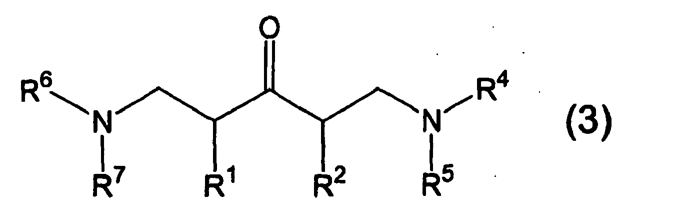
A₂) ein Enamin der allgemeinen Formel (1 a), worin R¹ und R² die vorstehend genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 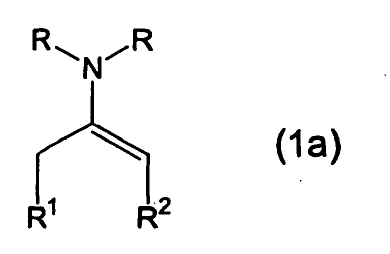 mit einem Aldehyd der allgemeinen Formel (4), worin R⁸ die vorstehend genannte Bedeutung mit Ausnahme von Wasserstoff hat, 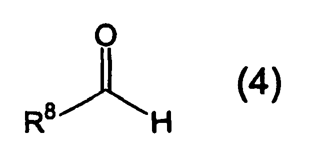 und einen Amin der allgemeinen Formel (2a), worin R⁴ und R⁵ die vorstehend genannte Bedeutung haben, gegebenenfalls in Form seines Hydrochlorids, 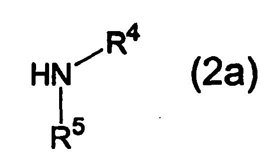 gemäß einer Mannich-Reaktion in Gegenwart von Triethylamin, Chlortrimethylsilan und Natriumiodid in einem geeigneten Lösungsmittel, vorzugsweise in Acetonitril, umsetzt, anschließend das Reaktionsgemisch aufarbeitet, das Keton der allgemeinen Formel (3a) isoliert und gegebenenfalls reinigt und 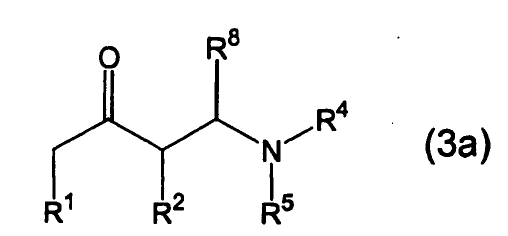 anschließend das Keton der allgemeinen Formel (3a) mit Paraformaldehyd und einem Amin der allgemeinen Formel (2), worin R⁶ und R⁷ die vorstehend genannte Bedeutung haben und wobei das Amin der allgemeinen Formel (2) vorzugsweise identisch mit dem Amin der allgemeinen Formel (2a) ist, 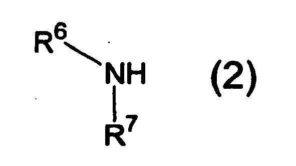 gemäß einer Mannich-Reaktion in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der Formel (3b) isoliert und gegebenenfalls reinigt oder 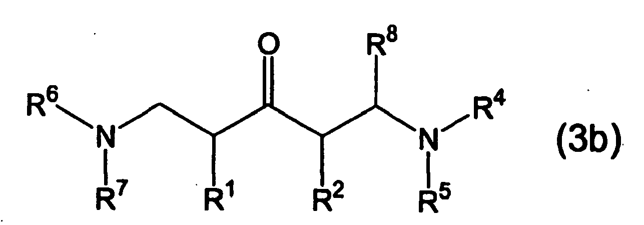
A₃) ein Enamin der allgemeinen Formel (1a), worin R¹ und R² die vorstehend genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 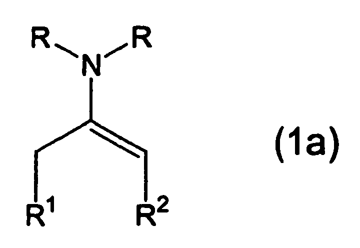 mit einem Iminiumsalz der allgemeinen Formel (5), worin R⁸ die vorstehend genannte Bedeutung mit Ausnahme von Wasserstoff und R⁴ und R⁵ die vorstehend genannte Bedeutung haben und Y⁻ für ein Chlorid-, Bromid-, Iodid- oder AlCl₄⁻-Ion steht, 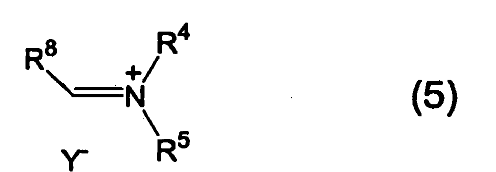 gemäß einer Mannich-Reaktion in einem geeigneten Lösungsmittel, vorzugsweise in Acetonitril, umsetzt, anschließend das Reaktionsgemisch aufarbeitet, das Keton der allgemeinen Formel (3a) isoliert und gegebenenfalls reinigt und 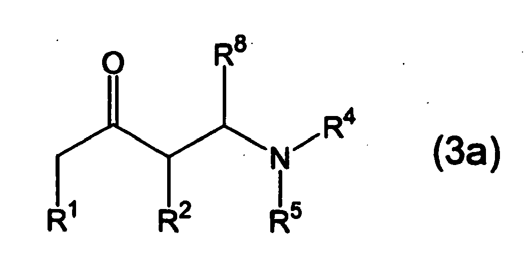 anschließend das Keton der allgemeinen Formel (3a) mit Paraformaldehyd und einem Amin der allgemeinen Formel (2), worin R⁶ und R⁷die vorstehend genannte Bedeutung haben und wobei das Amin der allgemeinen Formel (2) vorzugsweise identisch mit dem Amin der allgemeinen Formel (2a) ist, 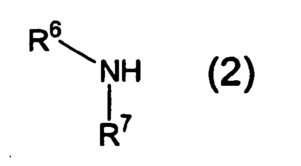 gemäß einer Mannich-Reaktion in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der allgemeinen Formel (3b) isoliert und gegebenenfalls reinigt und 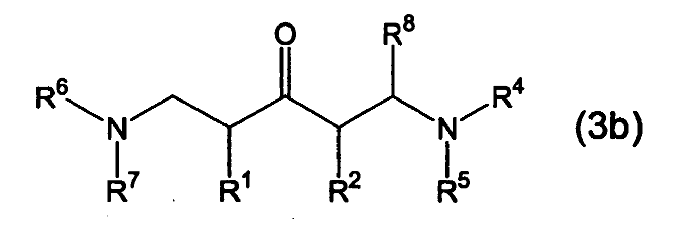
B) eine Verbindung der allgemeinen Formel (3) oder (3b) mit einer Grignard-Verbindung oder einer lithiumorganischen Verbindung der Formeln R³MgCl, R³MgBr, R³Mgl, MgR³₂ oder LiR³, worin R³ die vorstehend genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

Die verwendeten Ausgangsverbindungen sind am Markt erhältlich oder können nach üblichen dem Fachmann bekannten Methoden gewonnen werden.

Die Umsetzungen sind dem Fachmann aus der Literatur bekannt.

Die zum Einsatz kommenden Lösungsmittel und Umsetzungsbedindungen für die jeweilige Verfahrensstufe entsprechen den für diese Reaktionstypen üblichen Lösungsmitteln und Umsetzungsbedingungen.

Die freien Basen der jeweiligen erfindungsgemäßen Verbindungen der allgemeinen Formel I sowie entsprechender Stereoisomere können durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden. Bei den gebildeten Salzen handelt es sich unter anderem um Hydrochloride, Hydrobromide, Phosphate, Carbonate, Hydrogencarbonate, Formiate, Acetate, Oxalate, Succinate, Tartrate, Fumarate, Citrate und Glutaminate.

Die freien Basen der jeweiligen erfindungsgemäßen Verbindungen der allgemeinen Formel I sowie entsprechender Stereoisomere können durch Versetzen der in einem geeigneten organischen Lösungsmittel, wie z.B. Butan-2-on (Methylethylketon), gelösten erfindungsgemäßen Verbindungen der allgemeinen Formel I bzw. entsprechende Stereoisomere als freie Basen mit Trimethylsilylchlorid (TMSCI) in die entsprechenden Hydrochloride überführt werden. Entsprechend können sie auch in die Hydrobromide überführt werden.

Die freien Basen der jeweiligen erfindungsgemäßen Verbindungen der allgemeinen Formel I sowie entsprechender Stereoisomere können mit der freien Säure oder einem Salz eines Zuckerersatzstoffes, wie z. B. Saccharin, Cyclamat oder Acesulfam, in die entsprechenden physiologisch verträglichen Salze überführt werden.

Durch Kristallisation aus wäßriger Lösung können die Hydrate gebildet werden.

Sofern die erfindungsgemäßen Verbindungen der allgemeinen Formel I nach dem erfindungsgemäßen Herstellungsverfahren in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen Verbindungen der allgemeinen Formel I und entsprechende Stereoisomere sowie jeweils die entsprechenden Basen, Salze und Solvate sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße Verbindung der allgemeinen Formel I, vorzugsweise der allgemeinen Formel II, einschließlich der vorstehend ausgenommenen Verbindungen, in Form ihres Racemates, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates sowie gegebenenfalls physiologisch verträgliche Hilfsstoffe enthalten.

Sofern die erfindungsgemäßen Verbindungen der allgemeinen Formel I oder deren entsprechende physiologisch verträglichen Basen, Salze oder Solvate chiral sind, können sie - wie bereits ausgeführt - in Form ihrer reinen Enantiomeren, ihrer reinen Diastereomeren oder in Form eines Gemisches aus wenigstens zwei der vorstehend genannten Stereoisomeren - einschließlich ihrer Racemate - in dem erfindungsgemäßen Arzneimittel vorliegen.

Vorzugsweise eignen sich die erfindungsgemäßen Arzneimittel zur Bekämpfung von Schmerz, insbesondere von chronischem und/oder nichtchronischem Schmerz, als Lokalanästetikum, Antiarrhythmikum, Antiemetikum und/oder Nootropikum (Neurotropikum), zur Behandlung von inflammatorischen und/oder allergischen Reaktionen, cardiovaskulären Erkrankungen, Harninkontinenz, Diarrhöe, Gastritis, Ulcera, Schockzuständen, Migräne, Narkolepsie, Übergewicht, Asthma, Glaukom, Tinnitus, des hyperkinetischen Syndroms, Pruritus, Alkohol- und/oder Drogen- und/oder Medikamentenmißbrauch und/oder -abhängigkeit und/oder Entzündungen und/oder Depressionen und/oder zur Vigilanzsteigerung, zur Libidosteigerung und/oder zur Behandlung von neurodegenerativen Erkrankungen, insbesondere Morbus Parkinson und/oder Morbus Huntington, zur Behandlung und/oder Prophylaxe von Epilepsie, Schizophrenie, Morbus Alzheimer, Schlaganfall, cerebraler Ischämie, cerebralem Infarkt, Hirnödem und/oder zur Anxiolyse und/oder zur Anästhesie.

Die Verwendung wenigstens einer Verbindung der allgemeinen Formel I, vorzugsweise der allgemeinen Formel II, einschließlich der vorstehend ausgenommenen Verbindungen, in Form ihres Racemates, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates, zur Herstellung eines Arzneimittels zur Bekämpfung von Schmerz, insbesondere von chronischem und/oder nichtchronischem Schmerz, zur Lokalanästhesie, zur Behandlung von Arrhythmien, Emesis, inflammatorischen und/oder allergischen Reaktionen, cardiovaskulären Erkrankungen, Harninkontinenz, Diarrhöe, Gastritis, Ulcera, Schockzuständen, Migräne, Narkolepsie, Übergewicht, Asthma, Glaukom, Tinnitus, des hyperkinetischen Syndroms, Pruritus, Alkohol- und/oder Drogen- und/oder Medikamentenmißbrauch und/oder -abhängigkeit und/oder Entzündungen und/oder Depressionen und/oder zur Antriebssteigerung, Vigilanzsteigerung und/oder Libidosteigerung und/oder zur Behandlung von neurodegenerativen Erkrankungen, insbesondere Morbus Parkinson und/oder Morbus Huntington, zur Behandlung und/oder Prophylaxe von Epilepsie, Schizophrenie, Morbus Alzheimer, Schlaganfall, cerebraler Ischämie, cerebralem Infarkt, Himödem und/oder zur Anxiolyse und/oder zur Anästhesie ist ein weiterer Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, formuliert und als solche auch verabreicht werden.

Neben wenigstens einer erfindungsgemäßen Verbindung der allgemeinen Formel I, vorzugsweise der allgemeinen Formel II, einschließlich der vorstehend ausgenommenen Verbindungen, in Form ihres Racemates, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I, vorzugsweise der allgemeinen Formel II, einschließlich der vorstehend ausgenommenen Verbindungen, in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates, in einem Depot in gelöster Form oder in einem Pflaster, gegebenenfalls unter Zusatz von die Hautpenetration fördernden Mitteln, sind geeignete perkutane Applikationszubereitungen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen Verbindung der allgemeinen Formel I, vorzugsweise der allgemeinen Formel II, einschließlich der vorstehend ausgenommenen Verbindungen, in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer erfindungsgemäßen Verbindung der allgemeinen Formel I, vorzugsweise der allgemeinen Formel II, einschließlich der vorstehend ausgenommenen Verbindungen, in Form ihres Racemates, ihrer reinen Stereoisomeren, insbesondere Enantiomeren oder Diastereomeren, oder In Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates appliziert.

### Methode zur Bestimmung der Bindungsaffinität zum humanen alpha2A-adrenergen Rezeptor

Die Affinität der erfindungsgemäßen Verbindungen zum schmerzrelevanten alpha2A-Rezeptor wurde wie folgt untersucht.

Die Rezeptoraffinität der erfindungsgemäßen Verbindungen zum humanen alpha2A-adrenergen Rezeptor wurde in einem Mikrotiterplatten-Ansatz bestimmt. Hierzu wurden die zu prüfenden Verbindungen in einer Konzentration von 10 µmol/l mit einer Rezeptormembranpräparation aus humanen HT29-Zellen (RB-HAL2A, Fa. NEN, Zaventem, Belgien), welche den alpha2A-adrenergen Rezeptor endogen exprimieren, mit einer Proteinkonzentration von 40 µg Protein/250 µl Inkubationsansatz in Gegenwart von 0,5 nmol/l des radioaktiv markierten Liganden [3H]-MK-912 (NET-1059, Fa. NEN, Zaventem, Belgien) für 30 Minuten unter Lichtabschluß bei Raumtemperatur inkubiert. Als Puffersystem diente ein 25 mmol/l Natriumphosphatpuffer mit einem pH von 7,4. Die unspezifische Bindung wurde in Gegenwart von 10 µmol/l Phentolamin bestimmt. Nach der Inkubation wurden die Mikrotiterplatten auf Glasfaser-Mikrotiter-Filterplatten (Whatman GF/B, Fa. Hassel, München) mit einem Brandel Cell Harvester (Typ MPRI-96T, Fa. Hassel, München) gefiltert und nach Trocknen der Glasfaser-Filterplatten und anschließendem Beschicken der Platten mit 35 µl eines Szintillators (Ultima Gold, Fa. Canberra-Packard, Freiburg) in einem Mikrotiterplatten-Counter (1450 Microbeta Trilux, Fa. PerkinElmer-Wallac, Freiburg) nach einer mindestens 90 Minuten dauernden Verzögerung vermessen. Die Glasfaser-Mikrotiter-Filterplatten wurden vor der Filtration der Inkubationsplatten für 30 Minuten mit jeweils 50 µl je Vertiefung eines mit 0,5 % (v/v) Polyethylenimin supplementierten 25 mmol/l Natriumphosphatpuffer mit einem pH von 7,4 vorbehandelt. Die prozentuale Hemmwirkung der Verbindungen wurde als Verdrängung des radioaktiven Liganden aus seiner spezifischen Bindung zum humanen alpha2A-adrenergen Rezeptor berechnet.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die folgenden Beispiele zeigen die Darstellung der erfindungsgemäßen Verbindungen und die mit den erfindungsgemäßen Verbindungen durchgeführten Wirksamkeitsuntersuchungen.

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

### Allgemeine Synthesevorschriften zur Herstellung erfindungsgemäßer 1,5-Diaminopentan-3-ol-Verbindungen:

### Mannichreaktion I

0,1 Mol der jeweiligen Aminoverbindung der allgemeinen Formel (2a), 0,1 Mol Paraformaldehyd und 0,05 Mol der jeweiligen Ketoverbindung der allgemeinen Formel (1) wurden zusammen mit 20 ml Ethanol und 0.15 ml konzentrierter Salzsäure 6 Stunden unter Rückfluß erhitzt. Anschließend wurden 0,05 Mol Paraformaldehyd und 0,05 Mol der jeweiligen Aminoverbindung der allgemeinen Formel (2), wobei die Aminoverbindung der allgemeinen Formel (2) jeweils vorzugsweise identisch mit der Aminoverbindung der allgemeinen Formel (2a) ist, zugesetzt und weitere 10 Stunden unter Rückfluß erhitzt. Die gesamte Reaktionszeit betrug 16 Stunden. Anschließend wurde das Lösungsmittel im Vakuum abdestilliert, der Rückstand mit 50 ml Aceton versetzt und zum Auskristallisieren der Mannichverbindung der allgemeinen Formel (3) mehrere Tage bei +7°C stehen gelassen.

### Mannichreaktion II

Zu einer Natriumiodid-Lösung in Acetonitril (2,2 Äquivalente) wurde unter Eiskühlung die jeweilige Aminoverbindung der allgemeinen Formel (2a) (1Äquivalent) gegeben. Triethylamin (1Äquivalent), und Chlortrimethylsilan (2,2 Äquivalente) wurden zugetropft. Die Suspension wurde eine Stunde bei Raumtemperatur gerührt. Unter Eiskühlung wurde der jeweilige Aldehyd der allgemeinen Formel (4) (1 Äquivalent) zugegeben und eine Stunde bei Raumtemperatur gerührt. Ebenfalls unter Eiskühlung wurde 1 Äquivalent des jeweiligen Enamins zugegeben und zwei Stunden bei Raumtemperatur weitergerührt.

Der Ansatz wurde unter Eiskühlung mit verdünnter Salzsäure versetzt und 15 Minuten gerührt. Die Lösung wurde 3x mit Ether gewaschen. Mit verdünnter Ammoniak-Lösung wurde ein basischer pH-Wert eingestellt und mit Ether extrahiert. Nach dem Trocknen über Magnesiumsulfat wurde die etherische Phase enthaltend das gewünschte Produkt eingeengt.
Das Reaktionsprodukt der allgemeinen Formel (3a) wurde anschließend weiter umgesetzt.
0,1 Mol (1 Aquivalent) der Aminoverbindung der allgemeinen Formel (2), wobei die Aminoverbindung der allgemeinen Formel (2) vorzugsweise identisch mit der Aminoverbindung der allgemeinen Formel (2a) ist, 0,1 Mol Paraformaldehyd und 0,05 Mol (0,5 Äquivalente) des Reaktionsprodukts wurden zusammen mit 20 ml Ethanol und 0,15 ml konzentrierter Salzsäure 6 Stunden unter Rückfluß erhitzt. Anschließend wurde das Lösungsmittel im Vakuum abdestilliert, der Rückstand mit 50 ml Aceton versetzt und zum Auskristallisieren der Mannichverbindung der allgemeinen Formel (3b) mehrere Tage bei +7°C stehen gelassen.

### Grignardreaktion

In einem ausgeheizten und unter Inertgas auf -10°C abgekühlten Reaktionsgefäß wurde die in THF gelöste Mannichverbindung der jeweiligen allgemeinen Formel (3) oder (3b) (400µl, 0,5 M) vorgelegt. Unter Rühren wurden 2 Äquivalente des vorbereiteten Grignard- oder Organolithium-Reagenzes in THF oder Diethylether (800 µl, 0,5 M) zugegeben. Das Reaktionsgemisch wurde bei Raumtemperatur gerührt. Nach drei Stunden wurde erneut auf -10°C gekühlt und mit Ammoniumchlorid-Lösung hydrolisiert.

Das Reaktionsgemisch wurde zweimal mit Ethylacetat extrahiert und bei 40°C im Vakuum eingeengt.

Zur Charakterisierung der erfindungsgemäßen Verbindung der allgemeinen Formel I, vorzugsweise der allgemeinen Formel II, wurde jeweils ein ESI-MS aufgenommen.

### Bestimmung der Blndungsaffinität zum humanen alpha2A-adrenergen Rezeptor

Die Bindungsaffinitäten zum humanen alpha2A-adrenergen Rezeptor wurden gemäß der obenstehend angegebenen Methode bestimmt.

Die Werte einiger ausgewählter beispielgemäßer Verbindungen sind in der nachfolgenden Tabelle 1 wiedergegeben:

**Tabelle 1**

| Alpha2A, 10µM [%]-Hemmung | Erfindungsgemäße Verbindungen |
|---|---|
| 48 | 1-(3-Fluor-phenyl)-2,6-bis-piperidin-1-ylmethyl-cyclohexanol |
| 51 | 1-(3-Chlor-phenyl)-2,6-bis-piperidin-1-ylmethyl-cyclohexanol |
| 33 | 1-(3,5-Dichlor-phenyl)-2,6-bis-piperidin-1-ylmethyl-cyclohexanol |
| 100 | 1-(2-Chlor-benzyl)-2,6-bis-piperidin-1-ylmethyl-cyclohexanol |
| 87 | 1-(4-Fluor-benzyl)-2,6-bis-piperidin-1-ylmethyl-cyclohexanol |
| 80 | 1-(3-Methoxy-benzyl)-2,6-bis-piperidin-1-ylmethyl₋cyclohexanol |
| 26 | 1-(4-Chlor-3-trifluormethyl-phenyl)-2,6-bis-piperidin-1-ylmethylcyclohexanol |
| 59 | 1-(3-Fluor-benzyl)-2,6-bis-piperidin-1-ylmethyl-cyclohexanol |
| 30 | 1-(2-Methoxy-phenyl)-2,6-bis-piperidin-1-ylmethyl-cyclohexanol |
| 100 | 1-(2-Methyl-benzyl)-2,6-bis-piperidin-1-ylmethyl-cyclohexanol |
| 38 | 1-(3-Chlor-4-fluor-phenyl)-2,6-bis-piperidin-1-ylmethylcyclohexanol |
| 56 | 2,6-Bis-piperidin-1-ylmethyl-1-(3-trifluormethyl-phenyl)-cyclohexanol |
| 100 | 1-(3-Methyl-benzyl)-2,6-bis-piperidin-1-ylmethyl-cyclohexanol |
| 88 | 1-(4-Chlor-benzyl)-2,6-bis-piperidin-1-ylmethyl-cyclohexanol |
| 68 | 1-(2-Chlor-6-fluor-benzyl)-2,6-bis-piperidin-1-ylmethylcyclohexanol |
| 100 | 1-(2,5-Dimethyl-benzyl)-2,6-bis-piperidin-1-ylmethyl-cyclohexanol |
| 98 | 1-(3-Chlor-benzyl)-2,6-bis-piperidin-1-ylmethyl-cyclohexanol |
| 100 | 1-(2,4-Dichlor-benzyl)-2,6-bis-piperidin-1-ylmethyl-cyclohexanol |

## Patentansprüche

1. Substituierte 1,5-Diaminopentan-3-ol-Verbindungen der allgemeinen Formel I, 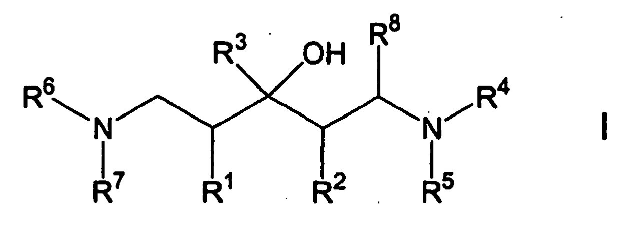 worin
R¹ und R², gleich oder verschieden, jeweils für einen linearen oder
verzweigten, gesättigten oder ungesättigten aliphatischen Rest stehen oder zusammen eine (CH₂)ₙ-Kette bilden, wobei n für eine ganze Zahl größer oder gleich 3 steht,
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert und/oder über eine lineare oder verzweigte, gesättigte oder ungesättigte aliphatische Brücke gebunden und/oder der Aryl- oder Heteroarylrest Teil eines polycyclischen Systems sein kann,
R⁴ und R⁵, gleich oder verschieden, jeweils für einen linearen oder verzweigten, gesättigten oder ungesättigten aliphatischen Rest oder einen über eine lineare oder verzweigte, gesättigte oder ungesättigte aliphatische Brücke gebundenen Arylrest stehen oder zusammen eine (CH₂)ₘ-Kette bilden, wobei m für eine ganze Zahl steht,
R⁶ und R⁷, gleich oder verschieden, jeweils für einen linearen oder verzweigten, gesättigten oder ungesättigten aliphatischen Rest oder einen über eine lineare oder verzweigte, gesättigte oder ungesättigte aliphatische Brücke gebundenen Arylrest stehen oder zusammen eine (CH₂)ₚ-Kette bilden, wobei p für eine ganze Zahl steht,
R⁸ für Wasserstoff oder einen gegebenenfalls einfach oder mehrfach substituierten Aryl- oder Heteroarylrest steht, wobei der Aryl- oder Heteroarylrest Teil eines polycyclischen Systems sein kann,
in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Basen oder in Form ihrer Salze, insbesondere der physiologisch verträglichen Salze, oder in Form ihrer Solvate, insbesondere der Hydrate,
mit Ausnahme der Verbindungen 1,5-Bis-(N,N'-dimethylamino)-2,4-dimethyl-3-pyridin-2-ylpentan-3-ol, 2,6-Bis-[(N,N'-dimethylamino)methyl]-1-phenylcyclohexanol, 2,6-Bis-[(N,N'-dimethylamino)methyl]-1-pyridin-2-ylcyclohexanol und 2,7-Bis-[(N,N'-dimethylamino)methyl]-1-pyridin-2-ylcycloheptanol.

2. Verbindungen gemäß Anspruch 1, **dadurch gekennzeichnet, daß**
R¹ und R², gleich oder verschieden, jeweils für einen linearen oder verzweigten, gesättigten oder ungesättigten aliphatischen C₁₋₆-Rest stehen oder zusammen eine (CH₂)ₙ-Kette bilden, wobei n für eine ganze Zahl von 3 - 9 steht,
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert und/oder über eine lineare oder verzweigte, gesättigte oder ungesättigte aliphatische C₁₋₅-Brücke gebunden sein kann,
R⁴ und R⁵, gleich oder verschieden, jeweils für einen linearen oder verzweigten, gesättigten oder ungesättigten aliphatischen C₁₋₆-Rest, einen über eine lineare oder verzweigte, gesättigte oder ungesättigte aliphatische C₁₋₅-Brücke gebundenen Phenylrest stehen oder zusammen eine (CH₂)ₘ-Kette bilden, wobei m für eine ganze Zahl von 4 - 10 steht,
R⁶ und R⁷, gleich oder verschieden, jeweils für einen linearen oder verzweigten, 2gesättigten oder ungesättigten aliphatischen C₁₋₆-Rest, einen über eine lineare oder verzweigte, gesättigte oder ungesättigte aliphatische C₁₋₅-Brücke gebundenen Phenylrest stehen oder zusammen eine (CH₂)ₚ-Kette bilden, wobei p für eine ganze Zahl von 4 - 10 steht,
R⁸ für Wasserstoff steht.

3. Verbindungen gemäß Anspruch 1, **dadurch gekennzeichnet, daß**
R¹ und R², gleich oder verschieden, jeweils für einen linearen oder verzweigten, gesättigten oder ungesättigten aliphatischen C₁₋₃-Rest stehen oder zusammen eine (CH₂)ₙ-Kette bilden, wobei n für eine ganze Zahl von 3 - 5 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann,
R⁴ und R⁵, gleich oder verschieden, jeweils für einen linearen oder verzweigten, gesättigten oder ungesättigten aliphatischen C₁₋₃-Rest stehen oder zusammen eine (CH₂)ₘ-Kette bilden, wobei m für eine ganze Zahl von 4 - 6 steht,
R⁶ und R⁷, gleich oder verschieden, jeweils für einen linearen oder verzweigten, gesättigten oder ungesättigten aliphatischen C₁₋₃-Rest stehen oder zusammen eine (CH₂)ₚ-Kette bilden, wobei p für eine ganze Zahl von 4 - 6 steht,
R⁸ für Wasserstoff steht.

4. Verbindungen gemäß Anspruch 3, **dadurch gekennzeichnet, daß** das jeweilige Ringsystem des Restes R³ gegebenenfalls einfach oder mehrfach mit Halogen, einer Alkylgruppe mit 1 bis 6 Kohlenstoffatomen, einer Alkoxygruppe mit 1 bis 6 Kohlenstoffatomen und/oder einer trihalogenierten Methylgruppe substituiert und/oder über eine lineare oder verzweigte, gesättigte oder ungesättigte aliphatische C₁₋₃-Brücke gebunden sein kann.

5. Verbindungen gemäß Anspruch 1, **dadurch gekennzeichnet, daß**
R¹ und R² zusammen eine (CH₂)ₙ-Kette bilden, wobei n für 3 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann,
R⁴ und R⁵ zusammen eine (CH₂)ₘ-Kette bilden, wobei m für 5 steht,
R⁶ und R⁷ zusammen eine (CH₂)ₚ-Kette bilden, wobei p für 5 steht,
R⁸ für Wasserstoff steht.

6. Verbindungen gemäß Anspruch 1 entsprechend der allgemeinen Formel II, 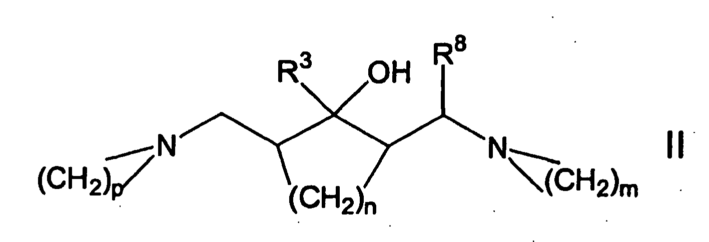 worin
m und p, gleich oder verschieden, für eine ganze Zahl von 4-10 stehen,
n für eine ganze Zahl größer oder gleich 3 steht.

7. Verbindungen gemäß Anspruch 6, **dadurch gekennzeichnet, daß**
m und p, gleich oder verschieden, für eine ganze Zahl von 4-10 stehen,
n für eine ganze Zahl von 3-9 steht und
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert und/oder über eine lineare oder verzweigte, gesättigte oder ungesättigte aliphatische C₁₋₅-Brücke gebunden sein kann,
R⁸ für Wasserstoff steht.

8. Verbindungen gemäß Anspruch 6, **dadurch gekennzeichnet, daß**
m und p, gleich oder verschieden, für eine ganze Zahl von 4-6 stehen,
n für eine ganze Zahl von 3-5 steht und
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert und/oder über eine lineare oder verzweigte, gesättigte oder ungesättigte aliphatische C₁₋₃-Brücke gebunden sein kann,
R⁸ für Wasserstoff steht.

9. Verbindungen gemäß Anspruch 8, **dadurch gekennzeichnet, daß** das jeweilige Ringsystem des Restes R³ gegebenenfalls einfach oder mehrfach mit Halogen, einer Alkylgruppe mit 1 bis 6 Kohlenstoffatomen, einer Alkoxygruppe mit 1 bis 6 Kohlenstoffatomen und/oder einer trihalogenierten Methylgruppe substituiert und/oder über eine lineare oder verzweigte, gesättigte oder ungesättigte, aliphatische C₁₋₃-Brücke gebunden sein kann.

10. Verbindungen gemäß Anspruch 6, **dadurch gekennzeichnet, daß**
m und p für 5 stehen,
n für 3 steht und
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert und/oder gegebenenfalls über eine lineare, gesättigte aliphatische C₁₋₃-Brücke oder eine Ethinyl-Brücke gebunden sein kann,und
R⁸ für Wasserstoff steht.

11. Eine substituierte 1,5-Diaminopentan-3-ol-Verbindung gemäß Anspruch 1 ausgewählt aus der Gruppe umfassend
1-Phenyl-2,6-bis-piperidin-1-ylmethyl-cyclohexanol,
1-(4-Chlor-phenyl)-2,6-bis-piperidin-1-ylmethyl-cyclohexanol,
1-Benzyl-2,6-bis-piperidin-1-ylmethyl-cyclohexanol,
1-(4-Fluor-3-methyl-phenyl)-2,6-bis-piperidin-1-ylmethyl-cyclohexanol,
2,6-Bis-piperidin-1-ylmethyl-1-o-tolyl-cyclohexanol,
2,6-Bis-piperidin-1-ylmethyl-1-vinyl-cyclohexanol,
1-(4-tert-Butyl-phenyl)-2,6-bis-piperidin-1-ylmethyl-cyclohexanol,
1-Cyclopentyl-2,6-bis-piperidin-1-ylmethyl-cyclohexanol,
2,6-Bis-piperidin-1-ylmethyl-1-m-tolyl-cyclohexanol,
2,6-Bis-piperidin-1-ylmethyl-bicyclohexyl-1-ol,
1-(4-Fluor-phenyl)-2,6-bis-piperidin-1-ylmethyl-cyclohexanol,
1-Phenethyl-2,6-bis-piperidin-1-ylmethyl-cyclohexanol,
1-Phenylethynyl-2,6-bis-piperidin-1-ylmethyl-cyclohexanol,
2,6-Bis-piperidin-1-ylmethyl-1-thiophen-2-yl-cyclohexanol,
1-(2,4-Dichlor-phenyl)-2,6-bis-piperidin-1-ylmethyl-cyclohexanol,
1-(3-Methoxy-phenyl)-2,6-bis-piperidin-1-ylmethyl-cyclohexanol,
1-(3-Phenyl-propyl)-2,6-bis-piperidin-1-ylmethyl-cyclohexanol,
1-(2,3-Dichlor-phenyl)-2,6-bis-piperidin-1-ylmethyl-cyclohexanol,
2,6-Bis-piperidin-1-ylmethyl-1-p-tolyl-cyclohexanol,
1-(4-Methoxy-phenyl)-2,6-bis-piperidin-1-ylmethyl-cyclohexanol,
1-Cyclohexylmethyl-2,6-bis-piperidin-1-ylmethyl-cyclohexanol,
1-(5-Fluor-2-methoxy-phenyl)-2,6-bis-piperidin-1-ylmethyl-cyclohexanol,
1-(3-Fluor-phenyl)-2,6-bis-piperidin-1-ylmethyl-cyclohexanol,
1-(3-Chlor-phenyl)-2,6-bis-piperidin-1-ylmethyl-cyclohexanol,
1-(3,5-Dichlor-phenyl)-2,6-bis-piperidin-1-ylmethyl-cyclohexanol,
1-(2-Chlor-benzyl)-2,6-bis-piperidin-1-ylmethyl-cyclohexanol,
1-(4-Fluor-benzyl)-2,6-bis-piperidin-1-ylmethyl-cyclohexanol,
1-(3-Methoxy-benzyl)-2,6-bis-piperidin-1-ylmethyl-cyclohexanol,
1-(4-Chlor3-trifluormethyl-phenyl)-2,6-bis-piperidin-1-ylmethyl-cyclohexanol,
1-(3-Fluor-benzyl)-2,6-bis-piperidin-1-ylmethyl-cyclohexanol,
1-(2-Methoxy-phenyl)-2,6-bis-piperidin-1-ylmethyl-cyclohexanol,
1-(2-Methyl-benzyl)-2,6-bis-piperidin-1-ylmethyl-cyclohexanol,
1-(3-Chlor-4-fluor-phenyl)-2,6-bis-piperidin-1-ylmethyl-cyclohexanol,
2,6-Bis-piperidin-1-ylmethyl-1-(3-trifluormethyl-phenyl)-cyclohexanol,
1-(3-Methyl-benzyl)-2,6-bis-piperidin-1-ylmethyl-cyclohexanol,
1-(4-Chlor-benzyl)-2,6-bis-piperidin-1-ylmethyl-cyclohexanol,
1-(2-Chlor-6-fluor-benzyl)-2,6-bis-piperidin-1-ylmethyl-cydohexanol,
1-(2,5-Dimethyl-benzyl)-2,6-bis-piperidin-1-ylmethyl-cyclohexanol,
1-(3-Chlor benryl)-2,6-bis-piperidin-1-ylmethyl-cyclohexanol und
1-(2,4-Dichlor-benzyl)-2,6-bis-piperidin-1-ylmethyl-cyclohexanol,
in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Base oder in Form ihres Salzes, insbesondere eines physiologisch verträglichen Salzes, oder in Form ihres Solvates, insbesondere des Hydrates.

12. Verfahren zur Herstellung von substituierten 1,5-Diaminopentan-3-ol-Verbindungen gemäß einem der Ansprüche 1-11, **dadurch gekennzeichnet, daß** man
A₁) ein Keton der Formel allgemeinen (1), worin R¹ und R² die in Anspruch 1 genannte Bedeutung haben, 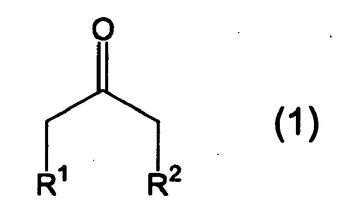 schrittweise mit Paraformaldehyd und jeweils einem Amin der allgemeinen Formel (2) oder (2a), worin R⁴, R⁵, R⁶ und R⁷die in Anspruch 1 genannte Bedeutung haben und wobei die Amine der allgemeinen Formeln (2) und (2a) vorzugsweise identisch sind, 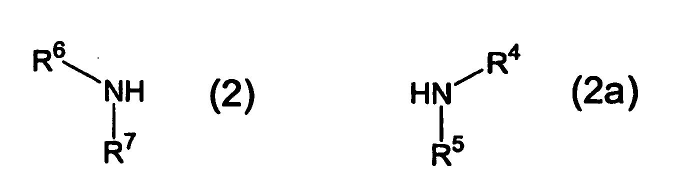 gemäß einer Mannich-Reaktlon in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der allgemeinen Formel (3) isoliert und gegebenenfalls reinigt oder 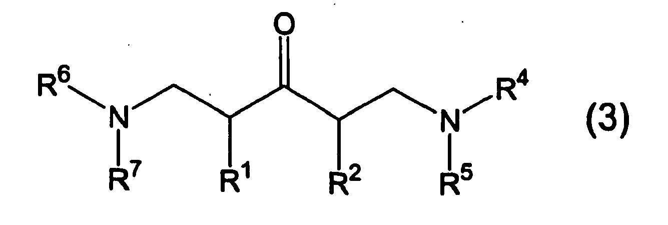
A₂) ein Enamin der allgemeinen Formel (1a), worin R¹ und R² die in Anspruch 1 genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 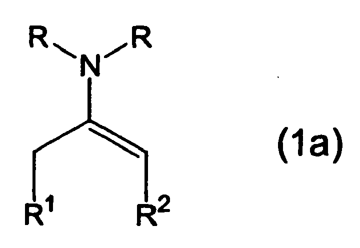 mit einem Aldehyd der allgemeinen Formel (4), worin R⁸ die in Anspruch 1 genannte Bedeutung mit Ausnahme von Wasserstoff hat, 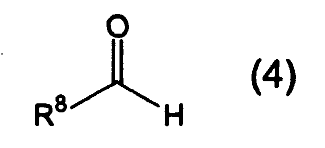 und einen Amin der allgemeinen Formel (2a), worin R⁴ und R⁵ die in Anspruch 1 genannte Bedeutung haben, gegebenenfalls in Form seines Hydrochlorids, 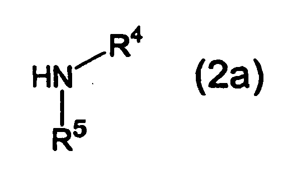 gemäß einer Mannich-Reaktion in Gegenwart von Triethylamin, Chlortrimethylsilan und Natriumiodid in einem geeigneten Lösungsmittel, vorzugsweise in Acetonitril, umsetzt, anschließend das Reaktionsgemisch aufarbeitet, das Keton der allgemeinen Formel (3a) isoliert und gegebenenfalls reinigt und 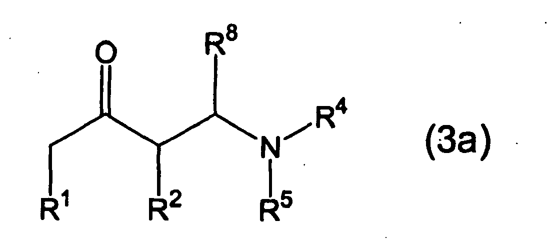 anschließend das Keton der allgemeinen Formel (3a) mit Paraformaldehyd und einem Amin der allgemeinen Formel (2), worin R⁶ und R⁷die in Anspruch 1 genannte Bedeutung haben und wobei das Amin der allgemeinen Formel (2) vorzugsweise identisch mit dem Amin der allgemeinen Formel (2a) ist, 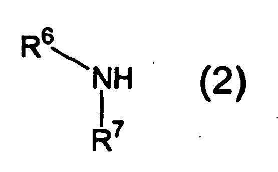 gemäß einer Mannich-Reaktion in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der allgemeinen Formel (3b) isoliert und gegebenenfalls reinigt oder 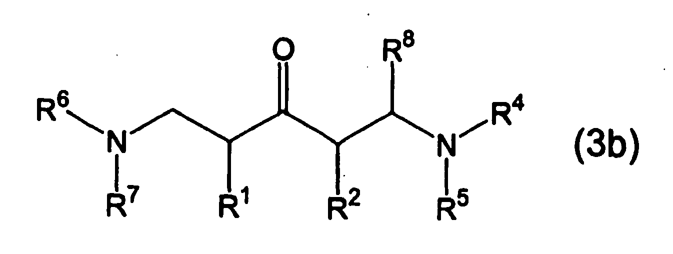
A₃) ein Enamin der allgemeinen Formel (1a), worin R¹ und R² die in Anspruch 1 genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 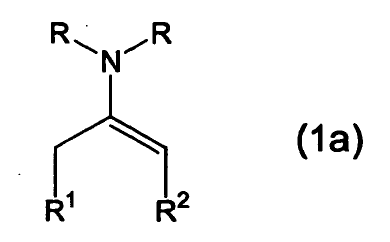 mit einem Iminiumsalz der allgemeinen Formel (5), worin R⁸ die in Anspruch 1 genannte Bedeutung mit Ausnahme von Wasserstoff und R⁴ und R⁵ die in Anspruch 1 genannte Bedeutung haben und Y⁻ für ein Chlorid-, Bromid-, Iodid- oder AlCl₄⁻-Ion steht, 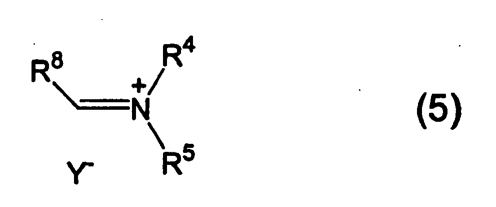 gemäß einer Mannich-Reaktion in einem geeigneten Lösungsmittel, vorzugsweise in Acetonitril, umsetzt, anschließend das Reaktionsgemisch aufarbeitet, das Keton der allgemeinen Formel (3a) isoliert und gegebenenfalls reinigt und 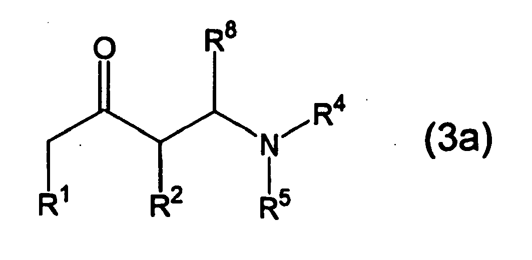 anschließend das Keton der allgemeinen Formel (3a) mit Paraformaldehyd und einem Amin der allgemeinen Formel (2), worin R⁶ und R⁷die in Anspruch 1 genannte Bedeutung haben und wobei das Amin der allgemeinen Formel (2) vorzugsweise identisch mit dem Amin der allgemeinen Formel (2a) ist, 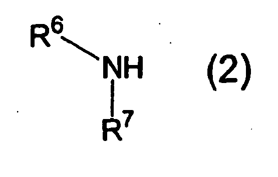 gemäß einer Mannich-Reaktion in einem geeigneten Lösungsmittel, vorzugsweise in Ethanol, unter Zusatz von Salzsäure oder in Essigsäure, unter Erhitzen umsetzt, anschließend das Reaktionsgemisch aufarbeitet, das Produkt der allgemeinen Formel (3b) isoliert und gegebenenfalls reinigt und 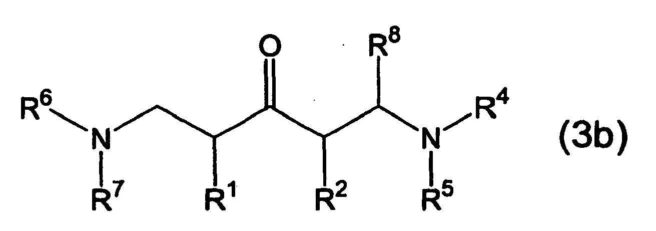
B) eine Verbindung der allgemeinen Formel (3) oder (3b) mit einer Grignard-Verbindung oder einer lithiumorganischen Verbindung der Formeln R³MgCl, R³MgBr, R³Mgl, MgR³₂ oder LiR³, worin R³ die in Anspruch 1 genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

13. Arzneimittel enthaltend wenigstens eine substituierte 1,5-Diaminopentan-3-ol-Verbindung gemäß einem der Ansprüche 1-11 und/oder 1,5-Bis-(N,N'-dimethylamino)-2,4-dimethyl-3-pyridin-2-ylpentan-3-ol und/oder 2,6-Bis-[(N,N'-dimethylamino)methyl]-1-phenylcyclohexanol und/oder 2,6-Bis-[(N,N'-dimethylamino)methyl]-1-pyridin-2-ylcyclohexanol und/oder 2,7-Bis-[(N,N'-dimethylamino)methyl]-1-pyridin-2-ylcycloheptanol als Wirkstoff und gegebenenfalls physiologisch verträgliche Hilfsstoffe.

14. Arzneimittel gemäß Anspruch 13 zur Bekämpfung von Schmerz.

15. Arzneimittel gemäß Anspruch 14 zur Bekämpfung von chronischem und/oder nichtchronischem Schmerz.

16. Arzneimittel gemäß Anspruch 13 als Lokalanästetikum.

17. Arzneimittel gemäß Anspruch 13 als Antiarrhythmikum.

18. Arzneimittel gemäß Anspruch 13 als Antiemetikum.

19. Arzneimittel gemäß Anspruch 13 als Nootropikum (Neurotropikum).

20. Arzneimittel gemäß Anspruch 13 zur Behandlung von inflammatorischen Reaktionen.

21. Arzneimittel gemäß Anspruch 13 zur Behandlung von allergischen Reaktionen.

22. Arzneimittel gemäß Anspruch 13 zur Behandlung von cardiovaskulären Erkrankungen.

23. Arzneimittel gemäß Anspruch 13 zur Behandlung von Harninkontinenz.

24. Arzneimittel gemäß Anspruch 13 zur Behandlung von Diarrhöe.

25. Arzneimittel gemäß Anspruch 13 zur Behandlung von Gastritis.

26. Arzneimittel gemäß Anspruch 13 zur Behandlung von Ulcera.

27. Arzneimittel gemäß Anspruch 13 zur Behandlung von Schockzuständen.

28. Arzneimittel gemäß Anspruch 13 zur Behandlung von Migräne.

29. Arzneimittel gemäß Anspruch 13 zur Behandlung von Narkolepsie.

30. Arzneimittel gemäß Anspruch 13 zur Behandlung von Übergewicht.

31. Arzneimittel gemäß Anspruch 13 zur Behandlung von Asthma.

32. Arzneimittel gemäß Anspruch 13 zur Behandlung von Glaukom.

33. Arzneimittel gemäß Anspruch 13 zur Behandlung von Tinnitus.

34. Arzneimittel gemäß Anspruch 13 zur Behandlung des hyperkinetischen Syndroms.

35. Arzneimittel gemäß Anspruch 13 zur Behandlung von Pruritus.

36. Arzneimittel gemäß Anspruch 13 zur Behandlung von Alkohol- und/oder Drogen- und/oder Medikamentenmißbrauch und/oder -abhängigkeit.

37. Arzneimittel gemäß Anspruch 13 zur Behandlung von Entzündungen.

38. Arzneimittel gemäß Anspruch 13 zur Behandlung von Depressionen.

39. Arzneimittel gemäß Anspruch 13 zur Vigilanzsteigerung.

40. Arzneimittel gemäß Anspruch 13 zur Libidosteigerung.

41. Arzneimittel gemäß Anspruch 13 zur Behandlung von neurodegenerativen Erkankungen, vorzugsweise Morbus Parkinson und Morbus Huntington.

42. Arzneimittel gemäß Anspruch 13 zur Behandlung und/oder Prophylaxe von Epilepsie.

43. Arzneimittel gemäß Anspruch 13 zur Behandlung und/oder Prophylaxe von Schizophrenie.

44. Arzneimittel gemäß Anspruch 13 zur Behandlung und/oder Prophylaxe von Morbus Alzheimer.

45. Arzneimittel gemäß Anspruch 13 zur Behandlung und/oder Prophylaxe von Schlaganfall.

46. Arzneimittel gemäß Anspruch 13 zur Behandlung und/oder Prophylaxe von cerebraler Ischämie.

47. Arzneimittel gemäß Anspruch 13 zur Behandlung und/oder Prophylaxe von cerebralem Infarkt.

48. Arzneimittel gemäß Anspruch 13 zur Behandlung und/oder Prophylaxe von Himödem.

49. Arzneimittel gemäß Anspruch 13 zur Anxiolyse.

50. Arzneimittel gemäß Anspruch 13 zur Anästhesie.

51. Verwendung wenigstens einer substituierten 1,5-Diaminopentan-3-ol-Verbindung gemäß einem der Ansprüche 1-11 und/oder 1,5-Bis-(N,N'-dimethylamino)-2,4-dimethyl-3-pyridin-2-ylpentan-3-ol und/oder 2,6-Bis-[(N,N'-dimethylamino)methyl]-1-phenylcyclohexanol und/oder 2,6-Bis-[(N,N'-dimethylamino)methyl]-1-pyridin-2-ylcyclohexanol und/oder 2,7-Bis-[(N,N'-dimethylamino)methyl]-1-pyridin-2-ylcycloheptanol zur Herstellung eines Arzneimittels zur Bekämpfung von Schmerz.

52. Verwendung wenigstens einer substituierten 1,5-Diaminopentan-3-ol-Verbindung gemäß einem der Ansprüche 1-11 und/oder 1,5-Bis-(N,N'-dimethylamino)-2,4-dimethyl-3-pyridin-2-ylpentan-3-ol und/oder 2,6-Bis-[(N,N'-dimethylamino)methyl]-1-phenylcyclohexanol und/oder 2,6-Bis-[(N,N'-dimethylamino)methyl]-1-pyridin-2-ylcyclohexanol und/oder 2,7-Bis-[(N,N'-dimethylamino)methyl]-1-pyridin-2-ylcycloheptanol zur Herstellung eines Arzneimittels zur Bekämpfung von chronischem und/oder nichtchronischem Schmerz.

53. Verwendung wenigstens einer substituierten 1,5-Diaminopentan-3-ol-Verbindung gemäß einem der Ansprüche 1-11 und/oder 1,5-Bis-(N,N'-dimethylamino)-2,4-dimethyl-3-pyridin-2-ylpentan-3-ol und/oder 2,6-Bis-[(N,N'-dimethylamino)methyl]-1-phenylcyclohexanol und/oder 2,6-Bis-[(N,N'-dimethylamino)methyl]-1-pyridin-2-ylcyclohexanol und/oder 2,7-Bis-[(N,N'-dimethylamino)methyl]-1-pyridin-2-ylcycloheptanol zur Herstellung eines Arzneimittels zur Lokalanästhesie, zur Behandlung von Arrhythmien, Emesis, inflammatorischen und/oder allergischen Reaktionen, cardiovaskulären Erkrankungen, Harninkontinenz, Diarrhöe, Gastritis, Ulcera, Schockzuständen, Migräne, Narkolepsie, Übergewicht, Asthma, Glaukom, Tinnitus, hyperkinetischem Syndrom, Pruritus, Alkohol- und/oder Drogen- und/oder Medikamentenmißbrauch und/oder-abhängigkeit und/oder Entzündungen, Depressionen und/oder zur Antriebssteigerung, Vigilanzsteigerung und/oder zur Libidosteigerung und/oder bei neurodegenerativen Erkankungen, vorzugsweise Morbus Parkinson und Morbus Huntington, und/oder zur Behandlung und/oder Prophylaxe von Epilepsie, Schizophrenie, Morbus Alzheimer, Schlaganfall, cerebraler Ischämie, cerebralem Infarkt und/oder Himödem und/oder zur Anxiolyse und/oder zur Anästhesie.

## Claims

1. Substituted 1,5-diaminopentan-3-ol compounds of the general formula I, 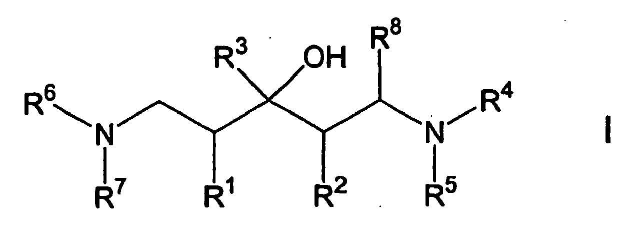 in which
R¹ and R², identical or different, in each case denote a linear or branched, saturated or unsaturated aliphatic residue, or together form a (CH₂)ₙ chain, wherein n denotes an integer of greater than or equal to 3,
R³ denotes a linear or branched, saturated or unsaturated aliphatic residue, a saturated or unsaturated cycloaliphatic residue, an aryl residue or a heteroaryl residue, wherein the respective ring system may optionally be mono- or polysubstituted and/or be attached via a linear or branched, saturated or unsaturated aliphatic bridge and/or the aryl or heteroaryl residue may be part of a polycyclic system,
R⁴ and R⁵, identical or different, in each case denote a linear or branched, saturated or unsaturated aliphatic residue or an aryl residue attached via a linear or branched, saturated or unsaturated aliphatic bridge or together form a (CH₂)ₘ chain, wherein m denotes an integer,
R⁶ and R⁷, identical or different, in each case denote a linear or branched, saturated or unsaturated aliphatic residue or an aryl residue attached via a linear or branched, saturated or unsaturated aliphatic bridge or together form a (CH₂)ₚ chain, wherein p denotes an integer, R⁸ denotes hydrogen or an optionally mono- or polysubstituted aryl or heteroaryl residue, wherein the aryl or heteroaryl residue may be part of a polycyclic system,
in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates,
with the exception of the compounds 1,5-bis-(N,N'-dimethylamino)-2,4-dimethyl-3-pyridin-2-ylpentan-3-ol, 2,6-bis-[(N,N'-dimethylamino)methyl]-1-phenylcyclohexanol, 2,6-bis-[(N,N'-dimethylamino)methyl]-1-pyridin-2-ylcyclohexanol and 2,7-bis-[(N,N,-dimethylamino)methyl]-1-pyridin-2-ylcycloheptanol.

2. Compounds according to claim 1, **characterised in that**
R¹ and R², identical or different, in each case denote a linear or branched, saturated or unsaturated aliphatic C₁₋₆ residue, or together form a (CH₂)ₙ chain, wherein n denotes an integer from 3-9,
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or be attached via a linear or branched, saturated or unsaturated aliphatic C₁₋₅ bridge,
R⁴ and R⁵, identical or different, in each case denote a linear or branched, saturated or unsaturated aliphatic C₁₋₆ residue, a phenyl residue attached via a linear or branched, saturated or unsaturated aliphatic C₁₋₅ bridge or together form a (CH₂)ₘ chain, wherein m denotes an integer from 4-10,
R⁶ and R⁷, identical or different, in each case denote a linear or branched, saturated or unsaturated aliphatic C₁₋₆ residue, a phenyl residue attached via a linear or branched, saturated or unsaturated aliphatic C₁₋₅ bridge or together form a (CH₂)ₚ chain, wherein p denotes an integer from 4-10,
R⁸ denotes hydrogen.

3. Compounds according to claim 1, **characterised in that**
R¹ and R², identical or different, in each case denote a linear or branched, saturated or unsaturated aliphatic C₁₋₃ residue, or together form a (CH₂)ₙ chain, wherein n denotes an integer from 3-5,
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or be attached via a linear or branched, saturated or unsaturated aliphatic C₁₋₃ bridge,
R⁴ and R⁵, identical or different, in each case denote a linear or branched, saturated or unsaturated aliphatic C₁₋₃ residue or together form a (CH₂)ₙ chain, wherein n denotes an integer from 4-6,
R⁶ and R⁷, identical or different, in each case denote a linear or branched, saturated or unsaturated aliphatic C₁₋₃ residue or together form a (CH₂)ₙ chain, wherein n denotes an integer from 4-6,
R⁸ denotes hydrogen.

4. Compounds according to claim 3, **characterised in that** the respective ring system of the residue R³ may optionally be mono- or polysubstituted with halogen, an alkyl group with 1 to 6 carbon atoms, an alkoxy group with 1 to 6 carbon atoms and/or a trihalogenated methyl group and/or be attached via a linear or branched, saturated or unsaturated aliphatic C₁₋₃ bridge.

5. Compounds according to claim 1, **characterised in that**
R¹ and R² together form a (CH₂)ₙ chain, wherein n denotes 3,
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge,
R⁴ and R⁵ together form a (CH₂)ₘ chain, wherein m denotes 5, R⁶ and R⁷ together form a (CH₂)ₚ chain, wherein p denotes 5,
R⁸ denotes hydrogen.

6. Compounds according to claim 1 corresponding to the general
formula II, 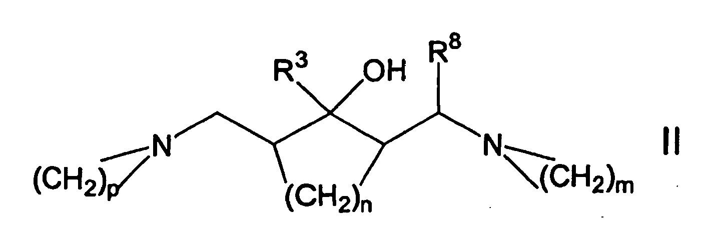 in which
m and p, identical or different, denote an integer from 4-10,
n denotes an integer greater than or equal to 3.

7. Compounds according to claim 6, **characterised in that**
m and p, identical or different, denote an integer from 4-10, n denotes an integer from 3-9 and
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or be attached via a linear or branched, saturated or unsaturated aliphatic C₁₋₅ bridge,
R⁸ denotes hydrogen.

8. Compounds according to claim 6, **characterised in that**
m and p, identical or different, denote an integer from 4-6,
n denotes an integer from 3-5 and
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or be attached via a linear or branched, saturated or unsaturated aliphatic C₁₋₃ bridge,
R⁸ denotes hydrogen.

9. Compounds according to claim 8, **characterised in that** the respective ring system of the residue R³ may optionally be mono- or polysubstituted with halogen, an alkyl group with 1 to 6 carbon atoms, an alkoxy group with 1 to 6 carbon atoms and/or a trihalogenated methyl group and/or be attached via a linear or branched, saturated or unsaturated, aliphatic C₁₋₃ bridge.

10. Compounds according to claim 6, **characterised in that** m and p denote 5,
n denotes 3 and
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge, and
R⁸ denotes hydrogen.

11. A substituted 1,5-diaminopentan-3-ol compound according to claim 1 selected from the group comprising
1-phenyl-2,6-bis-piperidin-1-ylmethylcyclohexanol,
1-(4-chlorophenyl)-2,6-bis-piperidin-1-ylmethylcyclohexanol,
1-benzyl-2,6-bis-piperidin-1-ylmethylcyclohexanol,
1-(4-fluoro-3-methylphenyl)-2,6-bis-piperidin-1-ylmethylcyclohexanol,
2,6-bis-piperidin-1-ylmethyl-1-o-tolylcyclohexanol,
2,6-bis-piperidin-1-ylmethyl-1-vinylcyclohexanol,
1-(4-tert-butylphenyl)-2,6-bis-piperidin-1-ylmethylcyclohexanol,
1-cyclopentyl-2,6-bis-piperidin-1-ylmethylcyclohexanol,
2,6-bis-piperidin-1-ylmethyl-1-m-tolylcyclohexanol,
2,6-bis-piperidin-1-ylmethylbicyclohexyl-1-ol,
1-(4-fluorophenyl)-2,6-bis-piperidin-1-ylmethylcydohexanol,
1-phenethyl-2,6-bis-piperidin-1-ylmethylcyclohexanol,
1-phenylethyl-2,6-bis-piperidin-1-ylmethylcyclohexanol,
2,6-bis-piperidin-1-ylmethyl-1-thiophen-2-ylcyclohexanol,
1-(2,4-dichlorophenyl)-2,6-bis-piperidin-1-ylmethylcyclohexanol,
1-(3-methoxyphenyl)-2,6-bis-piperidin-1-ylmethylcyclohexanol,
1-(3-phenylpropyl)-2,6-bis-piperidin-1-ylmethylcyclohexanol,
1-(2,3-dichlorophenyl)-2,6-bis-piperidin-1-ylmethylcyclohexanol,
2,6-bis-piperidin-1-ylmethyl-1-p-tolylcyclohexanol,
1-(4-methoxyphenyl)-2,6-bis-piperidin-1-ylmethylcyclohexanol,
1-cyclohexylmethyl-2,6-bis-piperidin-1-ylmethylcyclohexanol,
1-(5-fluoro-2-methoxyphenyl)-2,6-bis-piperidin-1-ylmethylcyclohexanol,
1-(3-fluorophenyl)-2,6-bis-piperidin-1-ylmethylcydohexanol,
1-(3-chiorophenyl)-2,6-bis-piperidin-1-ylmethylcyclohexanol,
1-(3,5-dichlorophenyl)-2,6-bis-piperidin-1-ylmethylcyclohexanol,
1-(2-chlorobenzyl)-2,6-bis-piperidin-1-ylmethylcyclohexanol,
1-(4-fluorobenzyl)-2,6-bis-piperidin-1-ylmethylcyclohexanol,
1-(3-methoxybenzyl)-2,6-bis-piperidin-1-ylmethylcyclohexanol,
1-(4-chloro-3-trifluoromethylphenyl)-2,6-bis-piperidin-1-ylmethylcyclohexanol,
1-(3-fluorobenzyl)-2,6-bis-piperidin-1-ylmethylcyclohexanol,
1-(2-methoxyphenyl)-2,6-bis-piperidin-1-ylmethylcyclohexanol,
1-(2-methylbenzyl)-2,6-bis-piperidin-1-ylmethylcyclohexanol,
1-(3-chloro-4-fluorophenyl)-2,6-bis-piperidin-1-ylmethylcyclohexanol,
2,6-bis-piperidin-1-ylmethyl-1-(3-trifluoromethylphenyl)-cyclohexanol,
1-(3-methylbenzyl)-2,6-bis-piperidin-1-ylmethylcyclohexanol,
1-(4-chlorobenzyl)-2,6-bis-piperidin-1-ylmethylcyclohexanol,
1-(2-chloro-6-fluorobenzyl)-2,6-bis-piperidin-1-ylmethylcyclohexanol,
1-(2,5-dimethylbenzyl)-2,6-bis-piperidin-1-ylmethylcyclohexanol,
1-(3-chlorobenzyl)-2,6-bis-piperidin-1-ylmethylcyclohexanol and
1-(2,4-dichlorobenzyl)-2,6-bis-piperidin-1-ylmethylcyclohexanol,
in the form the racemate thereof, the pure stereoisomers thereof, in particular enantiomer or diastereomer, or in the form of mixtures of the stereoisomers, in particular the enantiomers or diastereomers, in any desired mixing ratio, or in each case in the form of the base thereof or in the form of the salt thereof, in particular a physiologically acceptable salt, or in the form of the solvate thereof, in particular the hydrate.

12. A method for producing substituted 1,5-diaminopentan-3-ol compounds according to any one of claims 1-11, **characterised in that**
A₁) a ketone of the general formula (1), in which R¹ and R² have the meaning stated in claim 1, 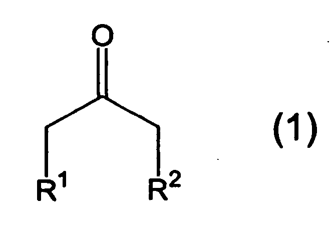 is reacted stepwise with paraformaldehyde and in each case an amine of the general formula (2) or (2a), in which R⁴, R⁵, R⁶ and R⁷ have the meaning stated in claim 1 and wherein the amines of the general formulae (2) and (2a) are preferably identical, 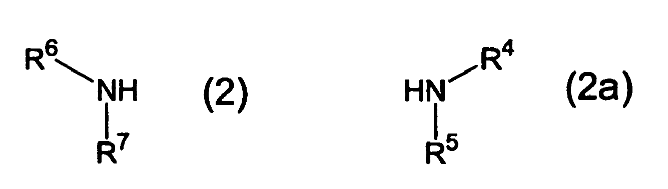 according to a Mannich reaction in a suitable solvent, preferably in ethanol with the addition of hydrochloric acid or in acetic acid, with heating, then the reaction mixture is worked up and the product of the general formula (3) is isolated and optionally purified or 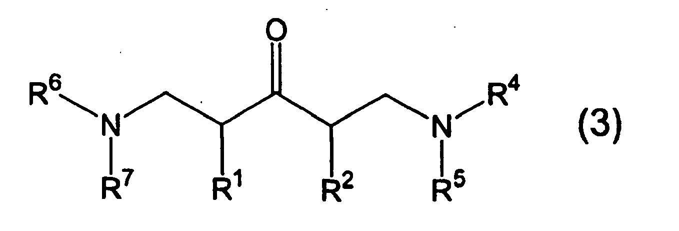
A₂) an enamine of the general formula (1a), in which R¹ and R² have the meaning stated in claim 1 and R denotes an aliphatic C₁₋₆ residue, a morpholinyl, piperidyl or pyrrolidinyl residue, wherein the two residues R may be identical or different, 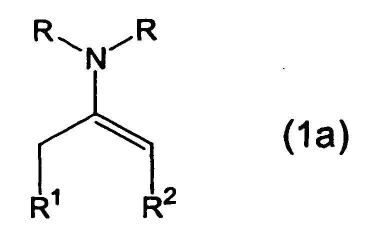 is reacted with an aldehyde of the general formula (4), in which R⁸ has the meaning stated in claim 1 with the exception of hydrogen, 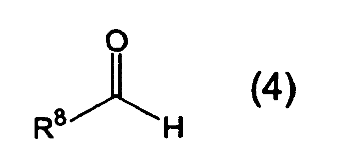 and an amine of the general formula (2a), in which R⁴ and R⁵ have the meaning stated in claim 1, optionally in the form of the hydrochloride thereof, 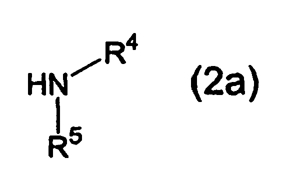 according to a Mannich reaction in the presence of triethylamine, chlorotrimethylsilane and sodium iodide in a suitable solvent, preferably in acetonitrile, then the reaction mixture is worked up and the product of the general formula (3a) is isolated and optionally purified and 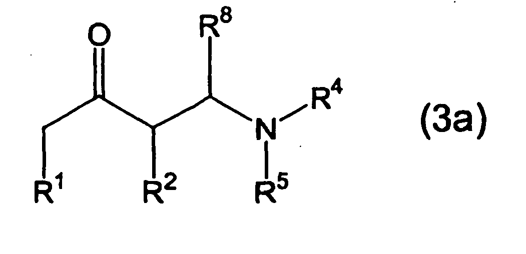 then the ketone of the general formula (3a) is reacted with paraformaldehyde and an amine of the general formula (2), in which R⁶ and R⁷ have the meaning stated in claim 1 and wherein the amine of the general formula (2) is preferably identical with the amine of the general formula (2a), 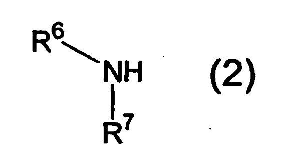 according to a Mannich reaction in a suitable solvent, preferably in ethanol, with the addition of hydrochloric acid or in acetic acid, with heating, then the reaction mixture is worked up and the product of the general formula (3b) is isolated and optionally purified or 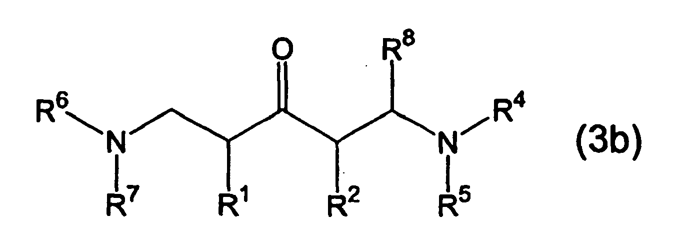
A₃) an enamine of the general formula (1a), in which R¹ and R² have the meaning stated in claim 1 and R denotes an aliphatic C₁₋₆ residue, a morpholinyl, piperidyl or pyrrolidinyl residue, wherein the two residues R may be identical or different, 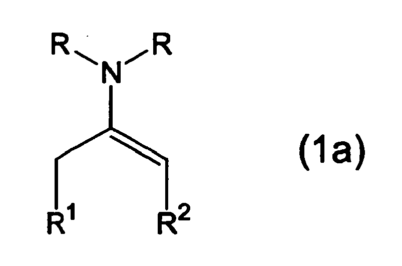 is reacted with an iminium salt of the general formula (5), in which R⁸ has the meaning stated in claim 1 with the exception of hydrogen and R⁴ and R⁵ have the meaning stated in claim 1 and Y⁻ denotes a chloride, bromide, iodide or AlCl₄⁻ ion, 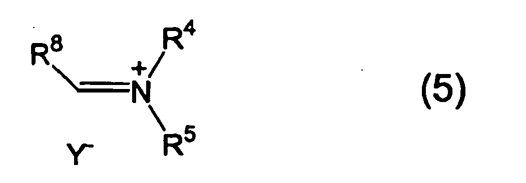 according to a Mannich reaction in a suitable solvent, preferably in acetonitrile, then the reaction mixture is worked up and the product of the general formula (3a) is isolated and optionally purified and 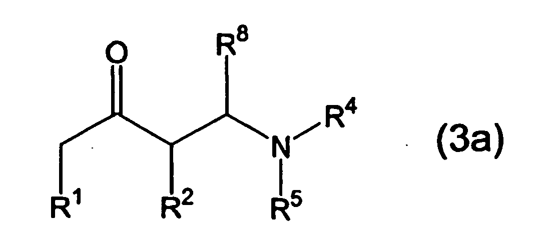 then the ketone of the general formula (3a) is reacted with paraformaldehyde and an amine of the general formula (2), in which R⁶ and R⁷ have the meaning stated in claim 1 and wherein the amine of the general formula (2) is preferably identical with the amine of the general formula (2a), 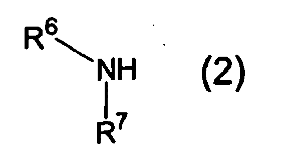 according to a Mannich reaction in a suitable solvent, preferably in ethanol with the addition of hydrochloric acid or in acetic acid, with heating, then the reaction mixture is worked up and the product of the general formula (3b) is isolated and optionally purified and 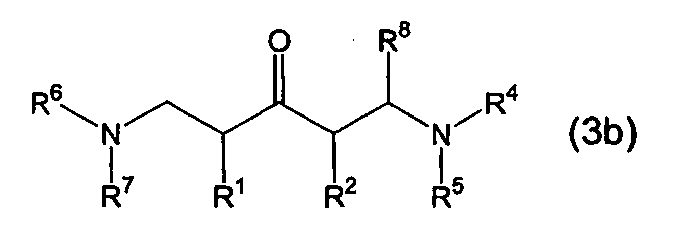
B) a compound of the formula (3) or (3a) is reacted with a Grignard compound or an organolithium compound of the formulae R³MgCl, R³MgBr, R³Mgl, MgR³₂ or LiR³, in which R³ has the meaning stated in claim 1, in a suitable solvent, preferably diethyl ether or tetrahydrofuran, then the reaction mixture is worked up and the compound of the general formula I is isolated and optionally purified.

13. A medicament containing at least one substituted 1,5-diaminopentan-3-ol compound according to any one of claims 1-11 and/or 1,5-bis-(N,N'-dimethylamino)-2,4-dimethyl-3-pyridin-2-ylpentan-3-ol and/or 2,6-bis-[(N,N'-dimethylamino)methyl]-1-phenylcyclohexanol and/or 2,6-bis-[(N,N'-dimethylamino)methyl]-1-pyridin-2-ylcyclohexanol and/or 2,7-bis-[(N,N'-dimethylamino)methyl]-1-pyridin-2-ylcycloheptanol as active ingredient and optionally physiologically acceptable auxiliary substances.

14. A medicament according to claim 13 for combating pain.

15. A medicament according to claim 14 for combating chronic and/or non-chronic pain.

16. A medicament according to claim 13 as an local anaesthetic.

17. A medicament according to claim 13 as an antiarrhythmic.

18. A medicament according to claim 13 as an antiemetic.

19. A medicament according to claim 13 as a nootropic (neurotropic).

20. A medicament according to claim 13 for the treatment of inflammatory reactions.

21. A medicament according to claim 13 for the treatment of allergic reactions.

22. A medicament according to claim 13 for the treatment of cardiovascular diseases.

23. A medicament according to claim 13 for the treatment of urinary incontinence.

24. A medicament according to claim 13 for the treatment of diarrhoea.

25. A medicament according to claim 13 for the treatment of gastritis.

26. A medicament according to claim 13 for the treatment of ulcers.

27. A medicament according to claim 13 for the treatment of shock states.

28. A medicament according to claim 13 for the treatment of migraine.

29. A medicament according to claim 13 for the treatment of narcolepsy.

30. A medicament according to claim 13 for the treatment of overweight.

31. A medicament according to claim 13 for the treatment of asthma.

32. A medicament according to claim 13 for the treatment of glaucoma.

33. A medicament according to claim 13 for the treatment of tinnitus.

34. A medicament according to claim 13 for the treatment of hyperkinetic syndrome.

35. A medicament according to claim 13 for the treatment of pruritus.

36. A medicament according to claim 13 for the treatment of the abuse of and/or dependency on alcohol and/or drugs and/or medicines.

37. A medicament according to claim 13 for the treatment of inflammation.

38. A medicament according to claim 13 for the treatment of depression.

39. A medicament according to claim 13 for increasing vigilance.

40. A medicament according to claim 13 for increasing libido.

41. A medicament according to claim 13 for the treatment of neurodegenerative diseases, preferably Parkinson's disease and Huntington's chorea.

42. A medicament according to claim 13 for the treatment and/or prevention of epilepsy.

43. A medicament according to claim 13 for the treatment and/or prevention of schizophrenia.

44. Medicament according to claim 13 for the treatment and/or prevention of Alzheimer's disease.

45. A medicament according to claim 13 for the treatment and/or prevention of stroke.

46. A medicament according to claim 13 for the treatment and/or prevention of cerebral ischaemia.

47. A medicament according to claim 13 for the treatment and/or prevention of cerebral infarct.

48. A medicament according to claim 13 for the treatment and/or prevention of cerebral oedema.

49. A medicament according to claim 13 for anxiolysis.

50. A medicament according to claim 13 for anaesthesia.

51. Use of at least one substituted 1,5-diaminopentan-3-ol compound according to any one of claims 1-11 and/or 1,5-bis-(N,N'-dimethylamino)-2,4-dimethyl-3-pyridin-2-ylpentan-3-ol and/or 2,6-bis-[(N,N'-dimethylamino)methyl]-1-phenylcyclohexanol and/or 2,6-bis-[(N,N'-dimethylamino)methyl]-1-pyridin-2-ylcyclohexanol and/or 2,7-bis-[(N,N'-dimethylamino)methyl]-1-pyridin-2-ylcycloheptanol for producing a medicament for combating pain.

52. Use of at least one substituted 1,5-diaminopentan-3-ol compound according to any one of claims 1-11 and/or 1,5-bis-(N,N-dimethylamino)-2,4-dimethyl-3-pyridin-2-ylpentan-3-ol and/or 2,6-bis-[(N,N'-dimethylamino)methyl]-1-phenylcyclohexanol and/or 2,6-bis-[(N,N'-dimethylamino)methyl]-1-pyridin-2-ylcyclohexanol and/or 2,7-bis-[(N,N'-dimethylamino)methyl]-1-pyridin-2-ylcycloheptanol for producing a medicament for combating chronic and/or non-chronic pain.

53. Use of at least one substituted 1,5-diaminopentan-3-ol compound according to any one of claims 1-11 and/or 1,5-bis-(N,N-dimethylamino)-2,4-dimethyl-3-pyridin-2-ylpentan-3-ol and/or 2,6-bis-[(N,N'-dimethylamino)methyl]-1-phenylcyclohexanot and/or 2,6-bis-[(N,N'-dimethylamino)methyl]-1-pyridin-2-ylcyclohexanol and/or 2,7-bis-[(N,N'-dimethylamino)methyl]-1-pyridin-2-ylcycloheptanol for producing a medicament for local anaesthesia, for the treatment of arrhythmias, emesis, inflammatory and/or allergic reactions, cardiovascular diseases, urinary incontinence, diarrhoea, gastritis, ulcers, shock states, migraine, narcolepsy, overweight, asthma, glaucoma, tinnitus, hyperkinetic syndrome, pruritus, abuse of and/or dependency on alcohol and/or drugs and/or medicines and/or inflammation, depression and/or for increasing drive, increasing vigilance and/or for increasing libido; and/or in neurodegenerative diseases, preferably Parkinson's disease and Huntington's chorea, and/or for treatment and/or prevention of epilepsy, schizophrenia, Alzheimer's disease, stroke, cerebral ischaemia, cerebral infarct and/or cerebral oedema and/or for anxiolysis and/or for anaesthesia.

## Revendications

1. Composés substitués de 1,5-diaminopentan-3-ol de formule générale I, 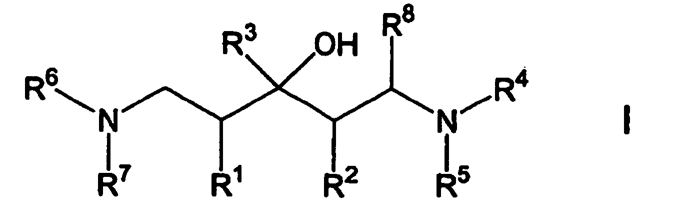 où
R¹ et R², identiques ou différents, représentent à chaque fois un radical aliphatique linéaire ou ramifié, saturé ou insaturé, ou forment ensemble une chaîne (CH₂)ₙ, où n représente un nombre supérieur ou égal à 3,
R³ représente un radical aliphatique linéaire ou ramifié, saturé ou insaturé, un radical cycloaliphatique saturé ou insaturé, un radical aryle ou un radical hétéroaryle, où le système cyclique peut le cas échéant être monosubstitué ou polysubstitué et/ou lié via un pont aliphatique linéaire ou ramifié, saturé ou insaturé et/ou la partie aryle ou hétéroaryle peut faire partie d'un système polycyclique,
R⁴ et R⁵, identiques ou différents, représentent à chaque fois un radical aliphatique linéaire ou ramifié, saturé ou insaturé, ou un radical aryle lié via un pont aliphatique linéaire ou ramifié, saturé ou insaturé ou forment ensemble une chaîne (CH₂)ₘ, où m représente un nombre entier,
R⁶ et R⁷, identiques ou différents, représentent à chaque fois un radical aliphatique linéaire ou ramifié, saturé ou insaturé, ou un radical aryle lié via un pont aliphatique linéaire ou ramifié, saturé ou insaturé ou forment ensemble une chaîne (CH₂)ₚ, où p représente un nombre entier,
R⁸ représente hydrogène ou un radical aryle ou hétéroaryle le cas échéant monosubstitué ou polysubstitué, où le radical aryle ou hétéroaryle peut faire partie d'un système polycyclique,
sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs bases ou sous forme de leurs sels, en particulier des sels physiologiquement acceptables, ou sous forme de leurs solvates, en particulier des hydrates,
à l'exception des composés 1,5-bis-(N,N'-diméthylamino)-2,4-diméthyl-3-pyridin-2-ylpentan-3-ol, 2,6-bis-[(N,N'-diméthylamino)méthyl]-1-phénylcyclohexanol, 2,6-bis-[(N,N'-diméthylamino)méthyl]-1-pyridin-2-ylcyclohexanol et 2,7-bis-[(N,N'-diméthylamino)méthyl]-1-pyridin-2-ylcycloheptanol.

2. Composés selon la revendication 1, **caractérisés en ce que**
R¹ et R², identiques ou différents, représentent à chaque fois un radical aliphatique en C₁₋₆ linéaire ou ramifié, saturé ou insaturé, ou forment ensemble une chaîne (CH₂)ₙ, où n représente un nombre entier de 3-9,
R³ représente un radical aliphatique en C₁₋₆ linéaire ou ramifié, saturé ou insaturé, un radical cycloaliphatique en C₃₋₇ saturé ou insaturé, un radical phényle ou un radical hétéroaryle de cinq ou six chaînons, où le système cyclique peut le cas échéant être monosubstitué ou polysubstitué et/ou lié via un pont aliphatique en C₁₋₅ linéaire ou ramifié, saturé ou insaturé,
R⁴ et R⁵, identiques ou différents, représentent à chaque fois un radical aliphatique en C₁₋₆ linéaire ou ramifié, saturé ou insaturé, un radical phényle lié via un pont aliphatique en C₁₋₅ linéaire ou ramifié, saturé ou insaturé ou forment ensemble une chaîne (CH₂)ₘ, où m représente un nombre entier de 4-10,
R⁶ et R⁷, identiques ou différents, représentent à chaque fois un radical aliphatique en C₁₋₆ linéaire ou ramifié, saturé ou insaturé, un radical phényle lié via un pont aliphatique en C₁₋₅ linéaire ou ramifié, saturé ou insaturé ou forment ensemble une chaîne (CH₂)ₚ, où p représente un nombre entier de 4-10,
R⁸ représente hydrogène.

3. Composés selon la revendication 1, **caractérisés en ce que**
R¹ et R², identiques ou différents, représentent à chaque fois un radical aliphatique en C₁₋₃ linéaire ou ramifié, saturé ou insaturé, ou forment ensemble une chaîne (CH₂)ₙ, où n représente un nombre entier de 3-5,
R³ représente un radical aliphatique en C₁₋₃ linéaire ou ramifié, saturé ou insaturé, un radical cycloaliphatique en C₅₋₆ saturé ou insaturé, un radical phényle ou un radical hétéroaryle de cinq ou six chaînons, où le système cyclique peut le cas échéant être monosubstitué ou polysubstitué par halogène, un groupe alkyle, un groupe alcoxy et/ou un groupe alkyle trihalogéné et/ou lié via un pont aliphatique en C₁₋₃ linéaire ou ramifié, saturé ou insaturé,
R⁴ et R⁵, identiques ou différents, représentent à chaque fois un radical aliphatique en C₁₋₃ linéaire ou ramifié, saturé ou insaturé, ou forment ensemble une chaîne (CH₂)ₘ, où m représente un nombre entier de 4-6,
R⁶ et R⁷, identiques ou différents, représentent à chaque fois un radical aliphatique en C₁₋₃ linéaire ou ramifié, saturé ou insaturé, ou forment ensemble une chaîne (CH₂)ₚ, où p représente un nombre entier de 4-6,
R⁸ représente hydrogène.

4. Composés selon la revendication 3, **caractérisés en ce que** le système cyclique du radical R³ peut le cas échéant être monosubstitué ou polysubstitué par halogène, un groupe alkyle comprenant 1 à 6 atomes de carbone, un groupe alcoxy comprenant 1 à 6 atomes de carbone et/ou un groupe méthyle trihalogéné et/ou être lié via un pont aliphatique en C₁₋₃ linéaire ou ramifié, saturé ou insaturé.

5. Composés selon la revendication 1, **caractérisés en ce que**
R¹ et R² forment ensemble une chaîne (CH₂)ₙ, où n représente 3,
R³ représente un radical vinyle, un radical cyclopentyle, un radical cyclohexyle, un radical thiophényle ou un radical phényle, où le radical cyclohexyle peut le cas échéant être lié via un pont méthylène ou le radical phényle peut le cas échéant être monosubstitué ou polysubstitué par fluor, chlore, un groupe méthyle, un groupe isopropyle, un groupe méthoxy et/ou un groupe trifluorométhyle et/ou peut le cas échéant être lié via un pont aliphatique en C₁₋₃ linéaire, saturé ou via un pont éthynyle,
R⁴ et R⁵ forment ensemble une chaîne (CH₂)_{m,} où m représente 5,
R⁶ et R⁷ forment ensemble une chaîne (CH₂)ₚ, où p représente 5,
R⁸ représente hydrogène.

6. Composés selon la revendication 1 correspondant à la formule générale II, 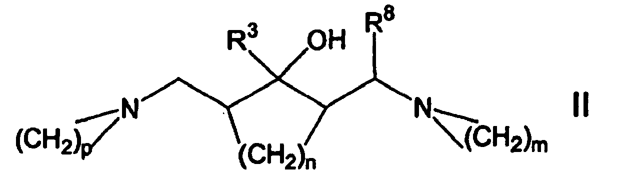 où
m et p, identiques ou différents, représentent un nombre entier de 4-10,
n représente un nombre entier supérieur ou égal à 3.

7. Composés selon la revendication 6, **caractérisés en ce que**
m et p, identiques ou différents, représentent un nombre entier de 4-10,
n représente un nombre entier de 3-9 et
R³ représente un radical aliphatique en C₁₋₆ linéaire ou ramifié, saturé ou insaturé, un radical cycloaliphatique en C₃₋₇ saturé ou insaturé, un radical phényle ou un radical hétéroaryle de cinq ou six chaînons, où le système cyclique peut le cas échéant être monosubstitué ou polysubstitué et/ou lié via un pont aliphatique en C₁₋₅ linéaire ou ramifié, saturé ou insaturé,
R⁸ représente hydrogène.

8. Composés selon la revendication 6, **caractérisés en ce que**
m et p, identiques ou différents, représentent un nombre entier de 4-6,
n représente un nombre entier de 3-5 et
R³ représente un radical aliphatique en C₁₋₃ linéaire ou ramifié, saturé ou insaturé, un radical cycloaliphatique en C₅₋₆ saturé ou insaturé, un radical phényle ou un radical hétéroaryle de cinq ou six chaînons, où le système cyclique peut le cas échéant être monosubstitué ou polysubstitué par halogène, un groupe alkyle, un groupe alcoxy et/ou un groupe alkyle trihalogéné et/ou lié via un pont aliphatique en C₁₋₃ linéaire ou ramifié, saturé ou insaturé,
R⁸ représente hydrogène.

9. Composés selon la revendication 8, **caractérisés en ce que** le système cyclique du radical R³ peut le cas échéant être monosubstitué ou polysubstitué par halogène, un groupe alkyle comprenant 1 à 6 atomes de carbone, un groupe alcoxy comprenant 1 à 6 atomes de carbone et/ou un groupe méthyle trihalogéné et/ou être lié via un pont aliphatique en C₁₋₃ linéaire ou ramifié, saturé ou insaturé.

10. Composés selon la revendication 6, **caractérisés en ce que**
m et p représentent 5,
n représente 3 et
R³ représente un radical vinyle, un radical cyclopentyle, un radical cyclohexyle, un radical thiophényle ou un radical phényle, où le radical cyclohexyle peut le cas échéant être lié via un pont méthylène ou le radical phényle peut le cas échéant être monosubstitué ou polysubstitué par fluor, chlore, un groupe méthyle, un groupe isopropyle, un groupe méthoxy et/ou un groupe trifluorométhyle et/ou peut le cas échéant être lié via un pont aliphatique en C₁₋₃ linéaire, saturé ou via un pont éthynyle, et
R⁸ représente hydrogène.

11. Composé substitué de 1,5-diaminopentan-3-ol selon la revendication 1, choisi dans le groupe comprenant les composés 1-phényl-2,6-bis-pipéridin-1-ylméthylcyclohexanol, 1-(4-chlorophényl)-2,6-bis-pipéridin-1-ylméthylcyclohexanol 1-benzyl-2,6-bis-pipéridin-1-ylméthylcyclohexanol 1-(4-fluoro-3-méthylphényl)-2,6-bis-pipéridin-1-ylméthylcyclohexanol 2,6-bis-pipéridin-1-ylméthyl-1-o-toluylcyclohexanol 2,6-bis-pipéridin-1-ylméthyl-1-vinylcyclohexanol 1-(4-tert-butylphényl)-2,6-bis-pipéridin-1-ylméthylcyclohexanol 1-cyclopentyl-2,6-bis-pipéridin-1-ylméthylcyclohexanol 2,6-bis-pipéridin-1-ylméthyl-1-m-toluylcyclohexanol 2,6-bis-pipéridin-1-ylméthylbicyclohexyl-1-ol 1-(4-fluorophényl)-2,6-bis-pipéridin-1-ylméthylcyclohexanol 1-phénéthyl-2,6-bis-pipéridin-1-ylméthylcyclohexanol 1-phényléthynyl-2,6-bis-pipéridin-1-ylméthylcyclohexanol 2,6-bis-pipéridin-1-ylméthyl-1-thiophén-2-ylcyclohexanol 1-(2,4-dichlorophényl)-2,6-bis-pipéridin-1-ylméthylcyclohexanol 1-(3-méthoxyphényl)-2,6-bis-pipéridin-1-ylméthylcyclohexanol 1-(3-phénylpropyl)-2,6-bis-pipéridin-1-ylméthylcyclohexanol 1-(2,3-dichlorophényl)-2,6-bis-pipéridin-1-ylméthylcyclohexanol 2,6-bis-pipéridin-1-ylméthyl-1-p-toluylcyclohexanol 1-(4-méthoxyphényl)-2,6-bis-pipéridin-1-ylméthylcyclohexanol 1-cyclohexylméthyl-2,6-bis-pipéridin-1-ylméthylcyclohexanol 1-(5-fluoro-2-méthoxyphényl)-2,6-bis-pipéridin-1-ylméthylcyclohexanol 1-(3-fluorophényl)-2,6-bis-pipéridin-1-ylméthylcyclohexanol 1-(3-chlorophényl)-2,6-bis-pipéridin-1-ylméthylcyclohexanol
1-(3,5-dichlorophényl)-2,6-bis-pipéridin-1-ylméthylcyclohexanol 1-(2-chlorobenzyl)-2,6-bis-pipéridin-1-ylméthylcyclohexanol 1-(4-fluorobenzyl)-2,6-bis-pipéridin-1-ylméthylcyclohexanol 1-(3-méthoxybenzyl)-2,6-bis-pipéridin-1-ylméthylcyclohexanol 1-(4-chloro-3-trifluorométhylphényl)-2,6-bis-pipéridin-1-ylméthylcyclohexanol 1-(3-fluorobenzyl)-2,6-bis-pipéridin-1-ylméthylcyclohexanol 1-(2-méthoxyphényl)-2,6-bis-pipéridin-1-ylméthylcyclohexanol 1-(2-méthylbenzyl)-2,6-bis-pipéridin-1-ylméthylcyclohexanol 1-(3-chloro-4-fluorophényl)-2,6-bis-pipéridin-1-ylméthylcyclohexanol 2,6-bis-pipéridin-1-ylméthyl-1-(3-trifluorométhylphényl)cyclohexanol 1-(3-méthylbenzyl)-2,6-bis-pipéridin-1-ylméthylcyclohexanol 1-(4-chlorobenzyl)-2,6-bis-pipéridin-1-ylméthylcyclohexanol 1-(2-chloro-6-fluorobenzyl)-2,6-bis-pipéridin-1-ylméthylcyclohexanol 1-(2,5-diméthylbenzyl)-2,6-bis-pipéridin-1-ylméthylcyclohexanol 1-(3-chlorobenzyl)-2,6-bis-pipéridin-1-ylméthylcyclohexanol et 1-(2,4-dichlorobenzyl)-2,6-bis-pipéridin-1-ylméthylcyclohexanol,
sous forme de ses racémates, ses stéréo-isomères purs, en particulier les énantiomères ou diastéréo-isomères, ou sous forme de mélanges des stéréo-isomères, en particulier des énantiomères ou des diastéréo-isomères, dans un rapport de mélange quelconque ou à chaque fois sous forme d'une base ou sous forme d'un sel, en particulier un sel physiologiquement acceptable, ou sous forme d'un solvate, en particulier de l'hydrate.

12. Procédé pour la préparation de composés substitués de 1,5-diaminopentan-3-ol selon l'une quelconque des revendications 1-11, **caractérisé en ce qu'**on transforme
A₁) une cétone de formule générale (1) dans laquelle R¹ et R² ont la signification mentionnée dans la revendication 1, 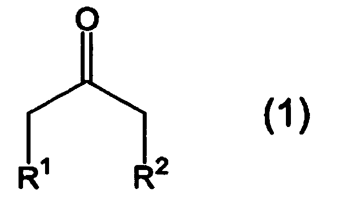 par étapes avec du paraformaldéhyde et à chaque fois une amine de formule générale (2) ou (2a), où R⁴, R⁵, R⁶ et R⁷ ont la signification mentionnée dans la revendication 1 et où les amines des formules générales (2) et (2a) sont de préférence identiques, 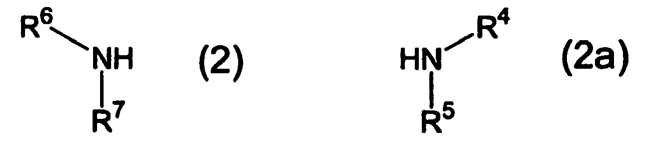 selon une réaction de Mannich, dans un solvant approprié, de préférence dans de l'éthanol, avec addition d'acide chlorhydrique ou dans de l'acide acétique, en chauffant, puis on traite le mélange réactionnel, on isole le produit de formule générale (3) et on le purifie le cas échéant ou 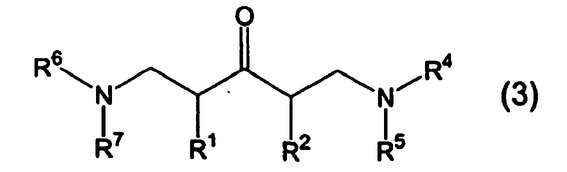
A₂) une énamine de formule générale (1a), où R¹ et R² ont la signification mentionnée dans la revendication 1 et R représente un radical aliphatique en C₁₋₆, un radical morpholinyle, pipéridyle ou pyrrolidinyle, où les deux radicaux R peuvent être identiques ou différents, 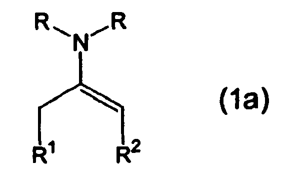 avec un aldéhyde de formule générale (4) dans laquelle R⁸ a la signification mentionnée dans la revendication 1, à l'exception de l'hydrogène, 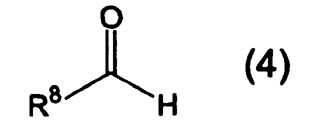 et une amine de formule générale (2a) dans laquelle R⁴ et R⁵ ont la signification mentionnée dans la revendication 1, le cas échéant sous forme de son chlorhydrate, 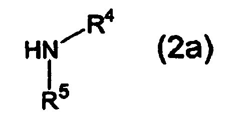 selon une réaction de Mannich en présence de triéthylamine, de chlorotriméthylsilane et d'iodure de sodium dans un solvant approprié, de préférence dans de l'acétonitrile, puis, on traite le mélange réactionnel, on isole la cétone de formule générale (3a) et on la purifie le cas échéant et 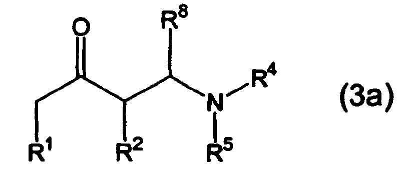 on transforme ensuite la cétone de formule générale (3a) avec du paraformaldéhyde et une amine de formule générale (2), où R⁶ et R⁷ ont la signification mentionnée dans la revendication 1 et où l'amine de formule générale (2) est de préférence identique à l'amine de formule générale (2a) 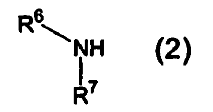 selon une réaction de Mannich, dans un solvant approprié, de préférence dans de l'éthanol, avec addition d'acide chlorhydrique ou dans de l'acide acétique, en chauffant, puis on traite le mélange réactionnel, on isole le produit de formule générale (3b) et on le purifie le cas échéant ou 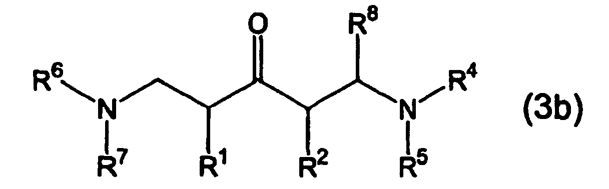
A₃) une énamine de formule générale (1a), où R¹ et R² ont la signification mentionnée dans la revendication 1 et R représente un radical aliphatique en C₁₋₆, un radical morpholinyle, pipéridyle ou pyrrolidinyle, où les deux radicaux R peuvent être identiques ou différents, 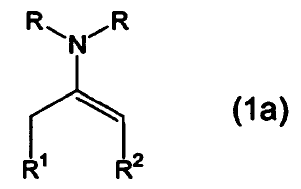 avec un sel d'iminium de formule générale (5), où R⁸ présente la signification mentionnée dans la revendication 1 à l'exception de l'hydrogène et R⁴ et R⁵ présentent la signification mentionnée dans la revendication 1 et Y⁻ représente un ion chlorure, bromure, iodure ou AlCl₄⁻, 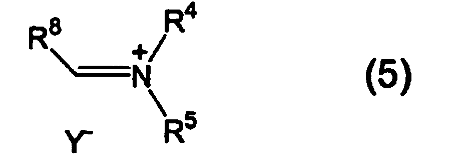 selon une réaction de Mannich dans un solvant approprié, de préférence dans de l'acétonitrile, puis, on traite le mélange réactionnel, on isole la cétone de formule générale (3a) et on la purifie le cas échéant et 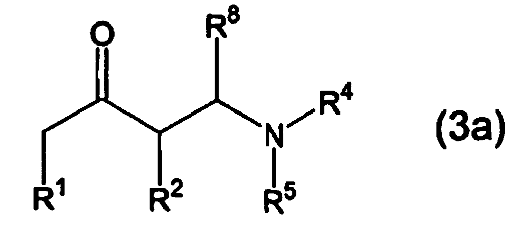 on transforme ensuite la cétone de formule générale (3a) avec du paraformaldéhyde et une amine de formule générale (2), où R⁶ et R⁷ ont la signification mentionnée dans la revendication 1 et où l'amine de formule générale (2) est de préférence identique à l'amine de formule générale (2a) 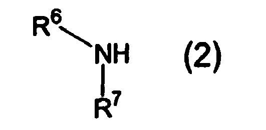 selon une réaction de Mannich, dans un solvant approprié, de préférence dans de l'éthanol, avec addition d'acide chlorhydrique ou dans de l'acide acétique, en chauffant, puis on traite le mélange réactionnel, on isole le produit de formule générale (3b) et on le purifie le cas échéant et 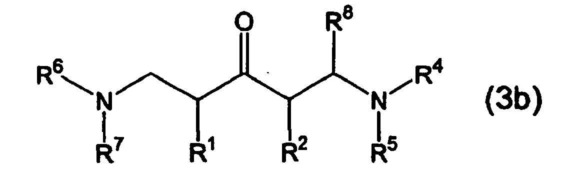
B) on transforme un composé de formule générale (3) ou (3b) avec un composé de Grignard ou un composé organique du lithium des formules R³MgCl, R³MgBr, R³MgI, MgR³2 ou LiR³, où R³ présente la signification mentionnée dans la revendication 1, dans un solvant approprié, de préférence du diéthyléther ou du tétrahydrofuranne, puis on traite le mélange réactionnel, on isole le composé de formule générale I et on le purifie le cas échéant.

13. Médicament contenant au moins un composé substitué du 1,5-diaminopentan-3-ol selon l'une quelconque des revendications 1-11 et/ou du 1,5-bis-(N,N'-diméthylamino)-2,4-diméthyl-3-pyridin-2-ylpentan-3-ol et/ou du 2,6-bis-[(N,N'-diméthylamino)méthyl]-1-phénylcyclohexanol et/ou du 2,6-bis-[(N,N'-diméthylamino)méthyl]-1-pyridin-2-ylcyclohexanol et/ou du 2,7-bis-[(N,N'-diméthylamino)méthyl]-1-pyridin-2-ylcycloheptanol comme substance active et le cas échéant des adjuvants physiologiquement acceptables.

14. Médicament selon la revendication 13 destiné à lutter contre la douleur.

15. Médicament selon la revendication 14 destiné à lutter contre la douleur chronique et/ou non chronique.

16. Médicament selon la revendication 13 comme anesthésique local.

17. Médicament selon la revendication 13 comme antiarythmique.

18. Médicament selon la revendication 13 comme antiémétique.

19. Médicament selon la revendication 13 comme nootropique (neurotropique).

20. Médicament selon la revendication 13 destiné au traitement de réactions inflammatoires.

21. Médicament selon la revendication 13 destiné au traitement de réactions allergiques.

22. Médicament selon la revendication 13 destiné au traitement de maladies cardiovasculaires.

23. Médicament selon la revendication 13 destiné au traitement de l'incontinence urinaire.

24. Médicament selon la revendication 13 destiné au traitement de la diarrhée.

25. Médicament selon la revendication 13 destiné au traitement de la gastrite.

26. Médicament selon la revendication 13 destiné au traitement d'ulcères.

27. Médicament selon la revendication 13 destiné au traitement d'états de choc.

28. Médicament selon la revendication 13 destiné au traitement de migraines.

29. Médicament selon la revendication 13 destiné au traitement de la narcolepsie.

30. Médicament selon la revendication 13 destiné au traitement de l'excès pondéral.

31. Médicament selon la revendication 13 destiné au traitement de l'asthme.

32. Médicament selon la revendication 13 destiné au traitement du glaucome.

33. Médicament selon la revendication 13 destiné au traitement des acouphènes.

34. Médicament selon la revendication 13 destiné au traitement du syndrome hyperkinétique.

35. Médicament selon la revendication 13 destiné au traitement du prurit.

36. Médicament selon la revendication 13 destiné au traitement de l'abus et/ou de la dépendance de l'alcool et/ou des drogues et/ou des médicaments.

37. Médicament selon la revendication 13 destiné au traitement d'inflammations.

38. Médicament selon la revendication 13 destiné au traitement de dépressions.

39. Médicament selon la revendication 13 destiné à l'augmentation de la vigilance.

40. Médicament selon la revendication 13 destiné à l'augmentation de la libido.

41. Médicament selon la revendication 13 pour le traitement de maladies de neurodégénérescence, de préférence la maladie de Parkinson et de Huntington.

42. Médicament selon la revendication 13 destiné au traitement et/ou à la prophylaxie de l'épilepsie.

43. Médicament selon la revendication 13 destiné au traitement et/ou à la prophylaxie de la schizophrénie.

44. Médicament selon la revendication 13 destiné au traitement et/ou à la prophylaxie de la maladie d'Alzheimer.

45. Médicament selon la revendication 13 destiné au traitement et/ou à la prophylaxie d'une attaque.

46. Médicament selon la revendication 13 destiné au traitement et/ou à la prophylaxie d'une ischémie cérébrale.

47. Médicament selon la revendication 13 destiné au traitement et/ou à la prophylaxie d'un infarctus cérébral.

48. Médicament selon la revendication 13 destiné au traitement et/ou à la prophylaxie d'un oedème cérébral.

49. Médicament selon la revendication 13 destiné à l'anxiolyse.

50. Médicament selon la revendication 13 destiné à l'anesthésie.

51. Utilisation d'au moins un composé substitué du 1,5-diaminopentan-3-ol selon l'une quelconque des revendications 1-11 et/ou du 1,5-bis-(N,N'-diméthylamino)-2,4-diméthyl-3-pyridin-2-ylpentan-3-ol et/ou du 2,6-bis-[(N,N'-diméthylamino)méthyl]-1-phénylcyclohexanol et/ou du 2,6-bis-[(N,N'-diméthylamino)méthyl]-1-pyridin-2-ylcyclohexanol et/ou du 2,7-bis-[(N,N'-diméthylamino)méthyl]-1-pyridin-2-ylcycloheptanol pour la préparation d'un médicament destiné à lutter contre la douleur.

52. Utilisation d'au moins un composé substitué du 1,5-diaminopentan-3-ol selon l'une quelconque des revendications 1-11 et/ou du 1,5-bis-(N,N'-diméthylamino)-2,4-diméthyl-3-pyridin-2-ylpentan-3-ol et/ou du 2,6-bis-[(N,N'-diméthylamino)méthyl]-1-phénylcyclohexanol et/ou du 2,6-bis-[(N,N'-diméthylamino)méthyl]-1-pyridin-2-ylcyclohexanol et/ou du 2,7-bis-[(N,N'-diméthylamino)méthyl]-1-pyridin-2-ylcycloheptanol pour la préparation d'un médicament destiné à lutter contre la douleur chronique et/ou non chronique.

53. Utilisation d'au moins un composé substitué du 1,5-diaminopentan-3-ol selon l'une quelconque des revendications 1-11 et/ou de 1,5-bis-(N,N'-diméthylamino)-2,4-diméthyl-3-pyridin-2-ylpentan-3-ol et/ou de 2,6-bis-[(N,N'-diméthylamino)méthyl]-1-phénylcyclohexanol et/ou de 2,6-bis-[(N,N'-diméthylamino)méthyl]-1-pyridin-2-ylcyclohexanol et/ou de 2,7-bis-[(N,N'-diméthylamino)méthyl]-1-pyridin-2-ylcycloheptanol pour la préparation d'un médicament destiné à l'anesthésie locale, au traitement d'arythmies, de l'émèse, de réactions inflammatoires et/ou allergiques, de maladies cardiovasculaires, de l'incontinence urinaire, de la diarrhée, de la gastrique, des ulcères, des états de choc, de la migraine, de la narcolepsie, de l'excès pondéral, de l'asthme, du glaucome, des acouphènes, du syndrome hyperkinétique, du prurit, de l'abus et/ou de la dépendance de l'alcool et/ou des drogues et/ou des médicaments et/ou des inflammations, des dépressions et/ou pour augmenter l'entrain, la vigilance et/ou la libido et/ou dans le cas de maladies de neurodégénérescence, de préférence la maladie de Parkinson et de Huntington, et/ou au traitement et/ou la prophylaxie de l'épilepsie, de la schizophrénie, de la maladie d'Alzheimer, de l'attaque, de l'ischémie cérébrale, de l'infarctus cérébral et/ou de l'oedème cérébral et/ou à l'anxiolyse et/ou à l'anesthésie.
